# FASCICULE DE BREVET EUROPEEN

(11) **EP 0 693 064 B1**
(45) Date de publication et mention de la délivrance du brevet: **14.01.1998**
(21) Numéro de dépôt: 94913149.4
(22) Date de dépôt: 08.04.1994
(51) Int. Cl.: C07D 311/16, C12Q 1/37

(54) **DERIVES D'AMINOCOUMARINES HYDROPHOBES, ET LEUR UTILISATION COMME SUBSTRATS D'ENZYMES PROTEOLYTIQUES OU POUR LA PREPARATION DE TELS SUBSTRATS**
HYDROPHOBE AMINOCOUMARIN-DERIVATE, IHRE VERWENDUNG ALS SUBSTRATE FÜR PROTEOLYTISCHE ENZYME, ODER ZUR HERSTELLUNG DIESER SUBSTRATE
HYDROPHOBIC AMINOCOUMARIN DERIVATIVES AND THEIR USE AS PROTEOLYTIC ENZYME SUBSTRATES OR IN THE PREPARATION OF SUCH SUBSTRATES

(30) Priorité: 08.04.1993 FR 9304190
(43) Date de publication de la demande: 24.01.1996
(73) Titulaire: Seratec, F-93800 Epinay-sur-Seine (FR)
(72) Inventeur: CHALOM, Joseph, F-75012 Paris (FR); LESUEUR, Pascal Bâtiment C6, F-95130 Franconville (FR); CHARLOT, Valérie, F-93200 Saint-Denis (FR); REVEILLEAU, Antoine, F-75006 Paris (FR)
(74) Mandataire: Des Termes, Monique
(86) Numéro de dépôt international: FR9400394
(87) Numéro de publication internationale: WO9424118

(56) Documents cités:
- EP-A- 0 247 373
- EP-A- 0 516 532
- WO-A-80/02295
- US-A- 4 897 444
- CHEMICAL ABSTRACTS, vol. 114, no. 21, 27 Mai 1991, Columbus, Ohio, US; abstract no. 206408v, & KHIM. GETEROTSIKL. SOEDIN., vol.12, 1990 pages 1600 - 1609 N.A. GORDEEVA ET AL. & CAS REGISTRY 1991 SUPPL. (STN DATABASE)
- HETEROCYCLES vol. 34, no. 2, 1992,, pages 273 - 291 T. BESSON ET AL.
- CHEMICAL ABSTRACTS, vol. 115, no. 7, 19 Aoüt 1991, Columbus, Ohio, US; abstract no. 63891e, & CHEMICAL AND PHARMACEUTICAL BULLETIN, vol.39, no.3, 1991, TOKYO JP pages 724 - 728 M. IRIKURA ET AL.
- CHEMICAL ABSTRACTS, vol. 104, no. 17, 28 Avril 1986, Columbus, Ohio, US; abstract no. 144508k, & INTER. J. PEPT. PROTEIN RES., vol.27, no.1, 1986 pages 7 - 17 D AMIR ET AL.
- DATABASE WPI Week 8010, Derwent Publications Ltd., London, GB; AN 80-16773C & DE,A,29 32 381 (AJINOMOTO K. K.) 28 Février 1980
- DATABASE WPI Week 7843, Derwent Publications Ltd., London, GB; AN 78-77582A & JP,A,53 108 974 (AJINOMOTO,K. K.) 22 Septembre 1978
- BIOL. CHEM. HOPPE-SEYLER, vol.366, Décembre 1985 pages 1113 - 1122 G. LEGLER ET AL. cité dans la demande
- CHEMICAL AND PHARMACEUTICAL BULLETIN, vol.25, no.2, Février 1977, TOKYO JP pages 362 - 363 Y. KANAOKA ET AL. cité dans la demande

## Description

La présente invention a pour objet de nouveaux dérivés d'aminocoumarines, utilisables in vitro comme substrats d'enzymes protéolytiques ou pour la préparation de tels substrats.

Les coumarines sont une importante famille de molécules présentant des applications très variées.

En effet, en dehors des domaines comme la pharmacologie où elles peuvent jouer le rôle d'anticoagulants, les coumarines sont très utilisées pour leurs propriétés optiques (fluorescence et chimiluminescence), par exemple en tant que
- colorants pour lasers,
- produits fluorescents pour l'optique,
- produits pour la synthèse de substrats d'enzymes utilisés dans des dosages d'enzymes, en particulier pour le diagnostic médical, et
- marqueur de molécules biologiques utiles également dans le domaine du diagnostic médical.

De façon plus précise, la présente invention concerne des dérivés d'aminocoumarines utilisables comme substrats d'enzymes protéolytiques ou pour la synthèse de tels substrats.

Pour la préparation d'un substrat d'enzyme protéolytique, on réalise le plus souvent une combinaison chimique entre a) un colorant ou un produit fluorescent et b) une molécule biochimique telle qu'un acide aminé, un peptide protégé ou non protégé, un acide carboxylique ou un acide gras.

Le substrat ainsi formé doit présenter les propriétés suivantes :
1°) les caractéristiques de coloration ou de fluorescence du colorant ou du produit fluorescent a) doivent être annulées ou très fortement déplacées ou diminuées dans le substrat,
2°) le substrat doit être capable de réagir avec des enzymes spécifiques pour subir un clivage,
3°) le clivage doit régénérer le produit colorant ou fluorescent a) et restaurer ainsi la couleur ou la fluorescence,
4°) cette restauration doit être proportionnelle à la quantité d'enzyme mise en oeuvre pour que les substrats puissent servir au dosage d'enzymes, et
5°) le substrat doit être non toxique pour pouvoir éventuellement être utilisé avec des cellules vivantes.

Parmi les dérivés d'aminocoumarines connus, on a utilisé jusqu'à présent pour la préparation de substrats d'enzymes protéolytiques, la 7-amino 4-méthyl coumarine, la 7-amino 4-trifluorométhyl coumarine, l'acide 7-amino coumarine 4-méthane sulfonique, la 4(3',6',9'-trioxanonyl) 7-amino coumarine, comme il est décrit dans Chem. Pharm. Bull. Vol. 25 1977, p. 362-363, dans J. Org. Chem., Vol. 45, p. 2283-2287, dans Chem. Pharm. Bull. Vol. 36, 1988, p. 3496-3502, et dans EP-A- 0 516 532.

Couplée à la L-alanine, la 7-amino-4-méthyl coumarine peut être utilisée par exemple comme substrat pour la L-alanine amino peptidase. Cette coumarine est très intéressante car elle est très fluorescente avec un maximum d'intensité de fluorescence, environ 14 fois plus élevé que celui des amines utilisées habituellement comme substrat, telles que la β-naphtylamine. Par ailleurs, il y a une différence d'intensité de fluorescence importante entre l'aminocoumarine et le substrat correspondant amide.

Le document EP-A-O 247 373 décrit un dérivé de coumarine de formule : 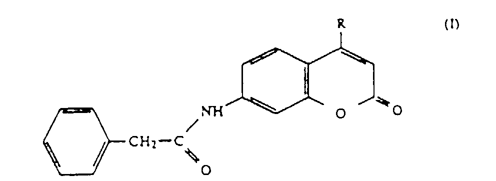 dans laquelle R est un groupe alkyle de 1 à 4, de préférence de 1 à 2 atomes de carbone, éventuellement substitué, en particulier le groupe méthyle ou trifluorométhyle.

Ce dérivé est un substrat fluorescent destiné à la pénicilline G-acylase.

Le document EP-A- 0 516 532 décrit en particulier des dérivés de coumarines hydrosolubles qui conviennent pour des dosages effectués en milieu homogène dans des solutions aqueuses où il est important d'avoir une bonne solubilité dans l'eau du substrat d'enzyme ou du colorant (a).

En revanche, ces dérivés de coumarines hydrosolubles ne conviennent pas pour détecter des réactions enzymatiques ayant lieu sur des supports solides en contact avec un milieu aqueux, par exemple pour la détection de réactions enzymatiques sur des coupes histologiques, sur des gels, papiers, etc. ... car dans ce cas, le colorant doit être hydrophobe pour ne pas être dissous ou élué mais maintenu précipité sur le site et y être détecté, visualisé ou dosé. En effet, avec les colorants hydrosolubles connus, ceux-ci diffuseront dans le milieu aqueux au lieu de rester sur le support solide et l'on ne pourra localiser sur le support les sites où se produit la réaction enzymatique par la coloration ou la fluorescence du colorant libéré.

Il en est de même, des autres dérivés connus de coumarine qui ont une hydrophilie trop importante : en raison de leur diffusion dans le milieu aqueux, ils ne permettront pas de visualiser ou quantifier les sites intéressants.

La présente invention a précisément pour objet de nouveaux dérivés d'aminocoumarines, utilisables in vitro comme substrats d'enzymes protéolytiques ou pour la préparation de tels substrats, qui présentent un caractère hydrophobe les rendant moins aptes à la diffusion dans une phase aqueuse, ce qui permet leur utilisation pour localiser des sites fluorescents sur des supports solides, en milieu hétérogène.

Selon l'invention, le dérivé d'aminocoumarine répond à la formule : 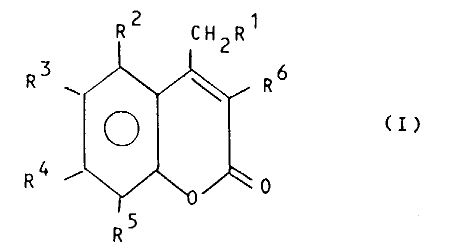 dans laquelle
1°) R¹ représente un groupe choisi parmi les groupes alkyle, linéaires ou ramifiés, en C₃ à C₁₇, les groupes cycloalkyle en C₃ à C₁₀ et les groupes aryle en C₆ à C₁₄, le groupe alkyle, cycloalkyle ou aryle étant non substitué ou substitué par au moins
   - un atome d'halogène,
2°) l'un des R², R³, R⁴ et R⁵ représente
   - NH₂
   - NHR⁷
   - NH-COOR⁷
   - N(R⁸)COOR⁷
   - NHR⁹, ou
   - NR⁷R⁹
   avec R⁷ et R⁸ identiques ou différents, représentant un groupe alkyle en C₁ à C₁₇, ou un groupe aryle en C₆ à C₁₄, ou avec R⁷ et R⁸ formant ensemble un cycle hydrocarboné, saturé ou insaturé, comportant éventuellement un hétéroatome choisi parmi 0, S, et N, et R⁹ représentant un groupe acyle, dérivé d'un composé choisi parmi les acides aminés, les peptides, les acides carboxyliques et les acides gras, le groupement amine des acides aminés ou des peptides étant protégé ou non protégé, salifié ou non salifié ;
3°) les autres R², R³, R⁴ et R⁵ qui peuvent être identiques ou différents, représentent
   - un atome d'hydrogène, ou
   - un groupe alkyle ou alcoxy, non substitué ou substitué par au moins un atome d'halogène, ou

   R² et R³, R³ et R⁴ ou R⁴ et R⁵ forment ensemble un cycle hydrocarboné saturé ou insaturé de 5 à 8 atomes de carbone, ou
   R³, R⁴ et R⁵, ou R², R³ et R⁴ forment ensemble avec le noyau phényle auquel ils sont liés un noyau polycyclique à 3 cycles condensés ; et
4°) R⁶ représente
   - un atome d'hydrogène, ou
   - un groupe choisi parmi les groupes alkyle en C₁ à C₁₇, aryle en C₆ à C₁₄ et cycloalkyle en C₃ à C₁₀, le groupe alkyle, aryle ou cycloalkyle étant non substitué ou substitué par au moins un atome d'halogène, et ses sels d'addition à des acides.

Dans le dérivé de coumarine de l'invention, la présence de R¹ qui est constitué par un groupe alkyle, un groupe cycloalkyle ou un groupe aryle, éventuellement substitué, confère au dérivé un caractère hydrophobe qui permet de limiter la diffusion de ce dérivé dans un milieu aqueux.

On a déjà réalisé des dérivés de coumarine comportant une chaîne hydrophobe comme il est décrit par Legler et al dans Biol. Chem. Hoppe - Seyler, vol. 366, p. 1113-1122, Décembre 1985. Cependant, dans ce cas, il s'agissait d'ombelliférone et non d'aminocoumarine ; de plus, selon ce document, la présence d'une chaîne hydrophobe a pour but de rendre le substrat enzymatique (ombelliféryl glucoside) plus spécifique d'une enzyme particulière, la glucosylcéramidase qui a une préférence pour les longues chaînes aliphatiques hydrophobes.

En revanche, dans l'invention, la présence d'un groupe R¹ ayant un caractère hydrophobe, a pour but d'empêcher la diffusion en phase aqueuse du colorant libéré par le substrat enzymatique sans modifier sa spécificité vis-à-vis de l'enzyme à laquelle il est destiné.

Selon l'invention, les groupes alkyle susceptibles d'être utilisés pour R¹ sont des groupes linéaires ou ramifiés ayant de 4 à 17 atomes de carbone, des groupes cycloalkyle de 3 à 10 atomes de carbone ou des groupes aryle de 6 à 14 atomes de carbone, ces groupes alkyle, cycloalkyle ou aryle pouvant être éventuellement substitués par au moins un atome d'halogène.

De préférence, dans l'invention, R¹ représente un groupe alkyle de 4 à 8 atomes de carbone, par exemple le groupe butyle ou pentyle.

Dans la formule (I) donnée ci-dessus, les groupes alkyle représentant R⁷, R⁸, R², R³, R⁴ et/ou R⁵ peuvent être des groupes linéaires ou ramifiés, ayant généralement de 1 à 10 atomes de carbone, de préférence 1 à 5 atomes de carbone.

Le terme "groupe alcoxy" utilisé pour R², R³, R⁴ et/ou R⁵, représente un groupe linéaire ou ramifié, ayant généralement de 1 à 10 atomes de carbone, de préférence 1 à 5 atomes de carbone.

Les termes "groupes aryle" utilisés pour R¹ à R⁸ désignent des radicaux dérivés d'hydrocarbures aromatiques comme le benzène et le naphtalène. A titre d'exemple de tels groupes, on peut citer Les groupes phényle et naphtyle.

Les termes "cycle hydrocarboné, saturé ou insaturé" désignent des hydrocarbures cycliques, saturés ou insaturés, ayant de 3 à 10 atomes de carbone. Lorsqu'ils comportent 1 ou plusieurs hétéroatomes, il peut s'agir par exemple de la pipéridine, de la pyridine, de la pipérazine, du pyrrole, du furanne, du pyranne et de la morpholine.

Dans le cas de R⁹, le groupe acyle peut être dérivé d'un acide aminé appartenant au groupe des acides aminés naturels, en particulier de ceux servant de substrats pour des enzymes. Ce groupe acyle peut être également dérivé d'un peptide formé de plusieurs acides aminés, en particulier d'acides aminés naturels.

Les acides aminés et les peptides peuvent se trouver sous la forme libre, sous la forme de sel, par exemple de chlorhydrate, ou sous une forme protégée, c'est-à-dire que le groupe amino terminal du peptide ou de l'acide aminé est protégé par un groupement approprié tel que ceux utilisés habituellement en synthèse peptidique, par exemple le groupe benzyloxycarbonyle (CBZ), le groupe t-butyloxycarbonyle (t-BOC), le groupe fluorénylméthoxycarbonyle (FmOC) ou le groupe succinyle.

Lorsque l'acide aminé ou le peptide formant R⁹ comportent d'autres groupes amino et/ou carboxyle libres, ceux-ci peuvent être protégés par des groupes appropriés ou être salifiés.

A titre d'exemple de peptides utilisables pour former le groupe acyle R⁹, on peut citer les peptides suivants : Pro-Arg ou tBOC-Val-Pro-Arg.

Le groupe acyle utilisé pour R⁹ peut encore être dérivé d'un acide carboxylique ou d'un acide gras ayant 4 à 18 atomes de carbone, utilisable comme substrat d'enzyme. A titre d'exemple de tels acides, on peut citer l'acide phénylacétique et l'acide butyrique.

Lorsque R² R³ et R⁴ ou R³, R⁴ et R⁵ forment avec le noyau phényle, un noyau polycyclique comportant éventuellement un ou plusieurs hétéroatomes, il peut s'agir par exemple du noyau suivant :

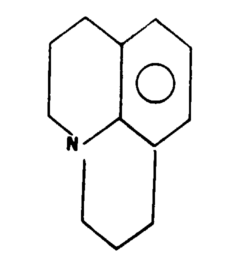

Selon un premier mode de réalisation de l'invention, les dérivés de coumarines de formule (I) sont destinés à être utilisés pour la synthèse de substrats d'enzymes. Dans ce cas, l'un des R², R³, R⁴ et R⁵, de préférence R⁴, représente -NH₂, -NHR⁷ ou -NH-COOR⁷, car on peut facilement coupler le dérivé de coumarine comportant un tel groupe à un composé approprié (acide aminé ou peptide) pour former un substrat d'enzyme.

Les dérivés conformes à ce premier mode de réalisation de l'invention constituent donc des produits intermédiaires très intéressants car, à partir d'un seul dérivé, on peut réaliser toute une gamme de substrats d'enzymes par réaction avec des composés appropriés.

Dans ce premier mode de réalisation, lorsque le groupe NH₂, NHR⁷ ou NH-COOR⁷ se trouve en position R⁴, R² R³, R⁵ et R⁶ représentent avantageusement un atome d'hydrogène.

Selon un second mode de réalisation de l'invention, les dérivés de coumarine sont destinés à être utilisés comme substrat d'enzyme. Dans ce cas, l'un des R², R³, R⁴ et R⁵ représente -NHR⁹ et R⁹ est choisi en fonction de l'enzyme à laquelle est destiné le substrat.

Ainsi, R⁹ peut être un radical dérivé de la L-alanine pour constituer un substrat de la L-alanine aminopeptidase, un radical dérivé de l'arginine, par exemple le groupe benzoxycarbonyl arginyle, pour constituer un substrat de la trypsine, le groupe pyroglutamyle pour former un substrat de la pyroglutanyl aminopeptidase ou le groupe γ-glutamyle pour former un substrat de la γ-glutamyltransférase. R⁹ peut encore être un radical acyle dérivé d'un peptide, par exemple Val-Pro-Arg protégé par le groupe butyloxycarbonyle, afin de servir de substrat pour la thrombine.

Dans ce second mode de réalisation, le groupe NHR⁹ ou NR⁷R⁹ est de préférence en position R⁴. Dans ce cas, R², R³, R⁵ et R⁶ représentent avantageusement un atome d'hydrogène.

Dans les deux modes de réalisation de l'invention, R⁶ représente de préférence un atome d'hydrogène, mais il peut aussi représenter un substituant permettant d'améliorer encore l'hydrophobie ou la fluorescence du dérivé.

Les dérivés de coumarine de l'invention peuvent être préparés par un procédé classique mettant en oeuvre la réaction de Pechman, à partir des esters β-cétoniques correspondants de formule :

R¹-CH₂-CO-CH(R⁶)-COOR¹⁰ (II)

dans laquelle R¹ et R⁶ ont les significations données ci-dessus, et R¹⁰ représente un groupe alkyle, un groupe aryle ou un groupe cycloalkyle.

En effet, en faisant réagir ces esters β-cétoniques comportant le groupement hydrophobe R¹, sur des phénols comportant les substituants R² à R⁵, on obtiendra le dérivé de coumarine de l'invention.

Les esters β-cétoniques de formule (II) peuvent être préparés par un procédé classique tel que celui décrit dans EP-A- 0 516 532, à partir des acides de formule R¹-CH₂-COOH.

Le procédé de préparation de ces esters β-cétoniques comprend, par exemple les étapes suivantes :
1°) transformer un acide de formule R¹-CH₂-COOH dans laquelle R¹ a la signification donnée ci-dessus, en l'imidazolide ou le chlorure d'acide correspondant,
2°) faire réagir l'imidazolide ou le chlorure d'acide avec un ester de formule : 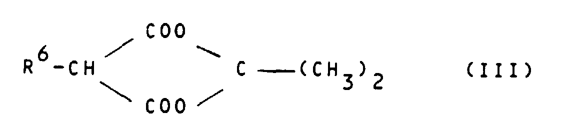 pour former un dérivé acylé, et
3°) faire réagir le dérivé acylé avec un alcool de formule R¹⁰OH pour obtenir l'ester β-cétonique de formule (II).

Les acides de départ R¹-CH₂-COOH ou les chlorures d'acide sont des produits du commerce ou peuvent être préparés par des procédés classiques.

L'ester de formule (III) existe dans le commerce (acide de Meldrum) ou peut être préparé par un procédé classique en partant de l'acide de Meldrum ou d'un malonate de diéthyle et en pratiquant deux substitutions successives sur le CH₂ malonique.

Pour préparer les dérivés de coumarines de formule (I) de l'invention, on fait réagir l'ester β-cétonique de formule (II) décrit ci-dessus avec un phénol de formule : 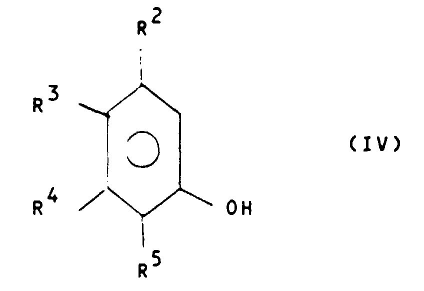 dans laquelle R², R³, R⁴ et R⁵ ont les significations données ci-dessus, l'un de ces substituants représentant NH₂, NHR⁷, NHCOOR⁷ ou NR⁸COOR⁷.

Cette réaction de condensation (réaction de Pechman) peut être effectuée dans l'eau, dans un alcool, dans un mélange mixte eau-alcool, en présence d'un catalyseur acide tel que H₂SO₄, HCl, CF₃COOH ou leurs sels, par exemple le chlorure d'aluminium, le chlorure de zinc ou le chlorure d'étain.

Une fois La condensation effectuée, le dérivé de coumarine sera purifié par les méthodes habituelles si la fonction amine est libre, c'est-à-dire Lorsque R⁴ représente NH₂ ou NHR⁷.

Si la fonction amine est protégée, c'est-à-dire que R⁴ représente NHCOOR⁷ ou NR⁸COOR⁷, elle sera déprotégée par hydrolyse en milieu acide ou par hydrogénolyse, puis le produit obtenu sera purifié par les méthodes classiques.

Pour préparer un dérivé de coumarine dans lequel l'un des groupes R², R³, R⁴ et R⁵ représente -NHR⁹ ou -NR⁷R⁹, on fait réagir une amino courmarine de formule (I) dans laquelle l'un des R², R³, R⁴ et R⁵ représente -NH₂ ou -NHR⁷, avec l'acide aminé, le peptide, l'acide carboxylique ou l'acide gras correspondant.

Pour cette réaction de couplage, le peptide ou l'acide aminé est généralement protégé sur sa fonction amine NH₂ par un groupement approprié tel que le groupe benzyloxycarbonyle ou le groupe t-butyloxycarbonyle.

Le couplage peut être effectué par une méthode classique de synthèse peptidique telle que celle décrite dans Bodanzki, Principles of Peptidic Synthesis, Springer Verlag, 1984, par exemple après formation d'un anhydride mixte (en utilisant le chloroformiate d'éthyle ou d'isobutyle, ou le chlorure de diphénylphosphinyle), ou par action d'un agent de condensation tel que le dicyclohexylcarbodiimide.

Une fois le couplage effectué, la fonction amine de l'acide aminé ou du peptide sera déprotégée par hydrogénation catalytique ou par hydrolyse acide. Le produit final étant éventuellement purifié par chromatographie, puis salifié.

Dans le cas de peptides, on peut coupler successivement le dérivé de coumarine comportant une fonction amine libre telle que NH₂ ou NHR⁷ avec les différents acides aminés formant le peptide.

Les dérivés de coumarines de formule (I) dans laquelle l'un des R², R³, R⁴ et R⁵ comprend un tel groupe acyle R⁹ peuvent être utilisés comme substrats d'enzymes dans un procédé de détection d'enzymes sur un support solide.

Pour cette détection, on peut utiliser le dérivé tel quel, ou sous la forme de chlorhydrate, bromhydrate, trifluoroacétate.

Pour effectuer cette détection, on prépare tout d'abord une solution, par exemple dans le DMSO de concentration choisie d'un dérivé de coumarine de formule (I) dans laquelle R² R³, R⁴, ou R⁵ représente NHR⁹ ou NR⁷R⁹, avec R⁹ pouvant jouer le rôle de substrat pour l'enzyme à détecter. On mélange cette solution au support solide (gel ...) ou on fait des dépôts de cette solution sur le support solide, par exemple coupes histologiques, frottis de cellules, papier ... contenant l'enzyme spécifique à détecter ou auquel l'enzyme spécifique à détecter sera ajouté, et on détermine après une durée choisie, par exemple par épifluorescence, la fluorescence ou la coloration du support mettant ainsi en évidence la présence d'enzyme spécifique sur le ou les sites fluorescents et la cinétique de la réaction.

Quand l'enzyme spécifique est apporté par des éléments figurés comme c'est le cas dans une coupe de tissu (biopsie), un frottis de cellules, des colonies de bactéries, des cultures de cellules il est ainsi possible de localiser, dans le tissu ou les colonies de cellules ou de bactéries, les cellules, les bactéries ou les organites de la cellule qui sont le site de l'activité enzymatique ; le colorant (a) hydrophobe n'étant pas dissous ou élue mais maintenu ou précipité sur place, restant ainsi localisé sur le site.

Enfin, ce test de détection peut être rendu quantitatif en mesurant la cinétique, l'intensité de la coloration ou de la fluorescence ou en la comparant à celle d'une gamme étalon obtenue dans le même temps, sur le même support solide, avec différentes quantités d'enzymes.

A titre d'exemple d'utilisation de ces dérivés de coumarine, on peut citer la différenciation des bactéries gram(+) et gram(-).

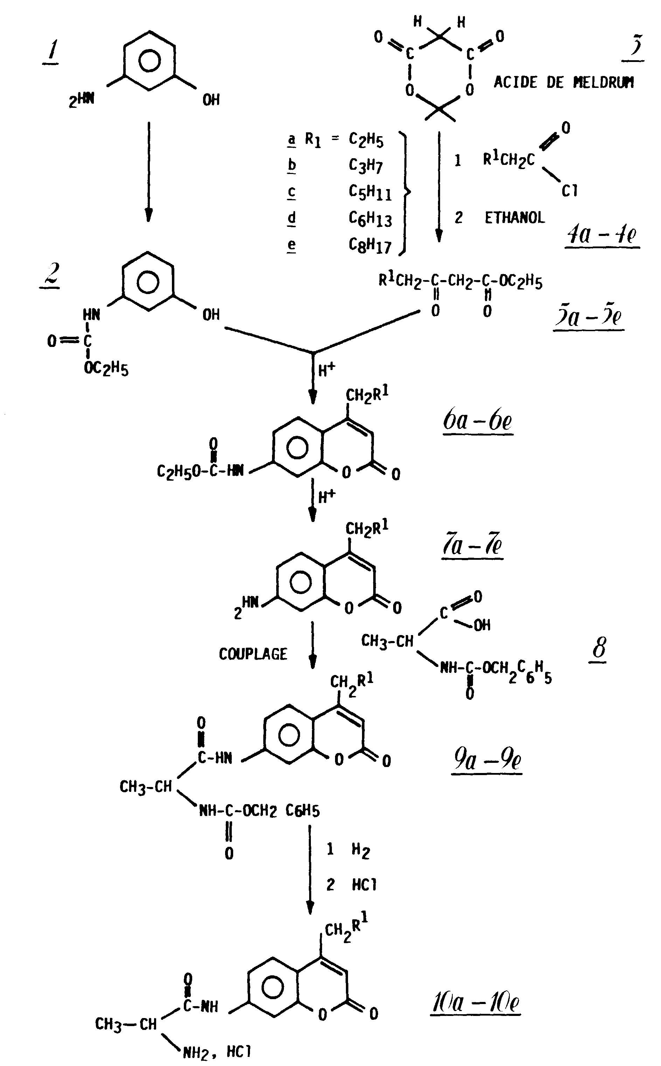

D'autres caractéristiques et avantage de l'invention apparaîtront mieux à la lecture des exemples suivants donnés bien entendu à titre illustratif et non limitatif en référence au dessin annexé.

Le dessin annexé est un schéma de préparation des dérivés de coumarine des exemples 1 à 15 avec R²=R³=R⁵=R⁶=H.

### Exemple 1 : Préparation de la 7-amino 4-propyl coumarine (7a)

Pour cette préparation, on condense le butyroyl acétate d'éthyle (**5a**) avec le 3-carbéthoxyaminophénol (**2**), en milieu acide, pour obtenir la 7-carbéthoxyamino 4-propyl coumarine (**6a**). On hydrolyse ensuite ce produit en milieu acéto-sulfurique pour obtenir la 4-propyl 7-amino coumarine (**7a**).

### a) Préparation de 3-carbéthoxy aminophénol (2)

On met sous agitation 109,13g (1mol) de 3-aminophénol **(1)** et 83g (1,05mol) de pyridine dans 500ml de dichlorométhane.

On introduit en 1h30, sous refroidissement, 109g (1mol) de chloroformiate d'éthyle en maintenant la température entre 10° et 15°C. On laisse ensuite 2h sous agitation.

La solution obtenue est lavée avec 200ml d'eau, 200ml d'acide chlorhydrique 1N et 200ml d'eau, séchée sur sulfate de magnésium et évaporée à sec.

Le concentrat obtenu (2) (150g ; Rt = 83%) est pur.

Les caractéristiques du produit sont les suivantes :
1°) C.C.M. (chromatographie en couche mince)
   silice fluorescente,
   dichlorométhane : méthanol (100:3),
   Révélation : UV longs et courts/iode/permanganate basique - monospot : Rf = 0,3.
2°) Spectre I.R. : 3300cm⁻¹ : υNH ; 3040cm⁻¹ : υCH aromatique ; 1690cm⁻¹ : υC=O uréthane ; 1615, 1580, 1505cm⁻¹ : υC=C aromatique ; 1280cm⁻¹ : υC-N ; 1250cm⁻¹ : υC-O phénol.

### b) Préparation de la 7-carbéthoxy-amino 4-propyl coumarine (6a)

On introduit sous agitation 0,67l d'acide sulfurique à 70%, 48g 10,3mol) de butyroyl acétate d'éthyle (**5a**), 50g (0,276mol) de 3-carbéthoxyaminophénol (**2**), et on laisse sous agitation durant 6h.

On coule la suspension formée sur 1kg de glace et on ajoute après 1/2h, 1,5l de dichlorométhane. On filtre le précipité qu'on lave au dichlorométhane et à l'eau. En concentrant à sec le dichlorométhane et en reprenant le résidu dans 200ml d'éthanol, on obtient un 2ième jet de produit (**6a**).

Rendement global = 42,5g (56%).

Les caractéristiques de ce produit sont les suivantes :
1°) C.C.M.
   Silice fluorescente,
   dichlorométhane/THF (85:2),
   révélation : UV courts et longs/iode,
   Rf = 0,4
2°) Spectre RMN (voir tableau 2)

### c) Préparation de la 7-amino 4-propyl coumarine (7a)

On met en suspension 27g (0,098mol) de 7-carbéthoxy-amino 4-propyl coumarine (**6a**) dans un mélange de 100g d'acide acétique et 100g d'acide sulfurique à 95%. On porte au reflux durant 4h.

On coule le mélange sur 400g de glace. En maintenant à 15-20°C, par refroidissement énergique, on ramène à pH 7,7 à l'aide de soude 12N (200mL). On filtre et on rince abondamment à l'eau. On recristallise le produit (**7a**) dans 120ml de butanol.

Rendement = 9,6g (48,2%).

Les caractéristiques du produit sont les suivantes :
1°) C.C.M.
   Silice fluorescente,
   dichlorométhane-méthanol (100:3),
   révélation : UV longs et courts/iode
   Rf = 0,5
2°) Spectre RMN (voir tableau n° 3)
3°) Spectre de masse (voir tableau n° 3).

### Exemple 2 : Préparation de la 7-amino 4-butyl coumarine (7b)

Pour cette préparation, on synthétise le valéroyl acétate d'éthyle (**5b**) par acylation de l'acide de Meldrum (**3**) suivie de l'éthanolyse de l'intermédiaire obtenu. On condense le valéroyl acétate d'éthyle (**5b**) avec le 3-carbéthoxyaminophénol (**2**), en milieu acide, pour obtenir la 7-carbéthoxyamino 4-butyl coumarine (**6b**). On hydrolyse ce produit en milieu acéto-sulfurique pour obtenir la 7-amino 4-butyl coumarine (**7b**).

### a) Préparation du valéroyl acétate d'éthyle (5b)

On introduit 189,3g (1,66mol) d'acide de Meldrum (**3**) dans 600ml de dichlorométhane anhydre. On ajoute en 1h 202,6g de 4-diméthylamino-pyridine à une température comprise entre 5° et 10°C.

On coule ensuite lentement, entre 5 et 10°C, une solution de 200g (1,66mol) de chlorure de valéroyle (**4b**) dans 400ml de dichlorométhane anhydre.

Après 1h d'agitation à 5°C et 1h à 20°C, on filtre le précipité formé que l'on élimine après rinçage. Le filtrat est lavé avec 21 d'acide chlorhydrique normal. La phase aqueuse est extraite avec 200ml de dichlorométhane. Les phases organiques sont lavées à l'eau, séchées sur sulfate de magnésium et évaporées à sec.

Le concentrat, repris par un litre d'éthanol est porté à reflux durant 2h.

Après évaporation sous vide, le résidu (218g) est distillé sous vide.

Rendement = 125g (44%)

Eb₅ₘₘ = 108-110°C.

Les caractéristiques du produit sont les suivantes.
1°) C.C.M.
   Silice fluorescente,
   heptane-acétate d'éthyle (70/30),
   révélation à l'iode (Rf = 0,7).
2°) Spectre de masse (voir tableau n° 1).

### b) Préparation de la 7-carbéthoxy-amino 4-butyl coumarine (6b)

On introduit sous agitation 1,32l d'acide sulfurique à 70%, 102,4g (0,59mol) de valéroyl acétate d'éthyle (**5b**), 98g (0,54mol) de carbéthoxyaminophénol (**2**), et on laisse sous agitation durant 8h.

On coule la suspension formée sur 1,5kg de glace et on ajoute après une demi-heure, 2l de dichlorométhane. On filtre le précipité qu'on rince au dichlorométhane et à l'eau. On sèche sous vide.

Rendement = 106g (68%).

Les caractéristiques du produit sont les suivantes.
1°) C.C.M.
   Silice fluorescente,
   dichlorométhane /THF (85:2),
   révélation : UV courts et longs,
   Rf=0,5
2°) Spectre RMN : (voir tableau n° 2).

### c) Préparation de la 7-amino 4-butyl coumarine (7b).

On met en suspension 100g (0,34mol) de 7-carbéthoxy-amino 4-butyl coumarine (**6b**) dans un mélange de 400ml d'acide acétique et 400ml d'acide sulfurique. On porte au reflux durant 8h.

On coule le mélange sur 1,5kg de glace et 400ml d'eau. En maintenant à 15-20°C par refroidissement énergique on ramène à pH8 à l'aide de soude 12N (1,3 l). On filtre et rince abondamment à l'eau. Après séchage, on recristallise le produit (78g) dans 1l d'éthanol.

Rendement = 42g (56%).

Les caractéristiques du produit sont les suivantes.
1°) C.C.M.
   Silice fluorescente,
   dichlorométhane : méthanol (100:3),
   révélation : UV courts et longs (Rf=0,8).
2°) F = 197-199°C
3°) Spectre RMN : (voir tableau n° 3)
4°) Spectre de masse : (voir tableau n° 3).

### Exemple 3 : Préparation de la 7-amino 4-hexyl coumarine (7c).

Pour cette préparation, on synthétise l'heptanoyl acétate d'éthyle (**5c**) en utilisant la même technique que dans l'exemple 2. On condense ce céto-ester avec le 3-carbéthoxyaminophénol (**2**), en milieu acide, pour obtenir la 7-carbéthoxy-amino 4-hexyl coumarine (**6c**). On hydrolyse ce produit en milieu acéto-sulfurique pour obtenir la 7-amino 4-hexyl coumarine (**7c**).

### a) Préparation de l'heptanoyl acétate d'éthyle (5c).

On introduit 285g (2,5mol) d'acide de Meldrum (**3**) dans 900ml de dichlorométhane anhydre. On ajoute en 1h 305g (2,5mol) de 4-diméthylaminopyridine à une température comprise entre 5 et 10°C.

On coule ensuite lentement entre 5 et 10°C, une solution de 371g (2,5mol) de chlorure d'heptanoyle (**4c**) dans 950ml de dichlorométhane anhydre.

Après 1h d'agitation à 5°C, et 1h à 20°C, on filtre le précipité formé que l'on élimine après rinçage. Le filtrat est lavé avec 2l d'acide chlorhydrique normal. La phase aqueuse est extraite avec 500ml de dichlorométhane. Les phases organiques sont lavées à l'eau, séchées sur sulfate de magnésium et évaporées à sec.

Le concentrat, repris par 3l d'éthanol, est porté à reflux durant 2h. On évapore l'éthanol à sec, et on utilise le produit tel quel pour le stade suivant.

Les caractéristiques du produit sont les suivantes.
1°) C.C.M.
   Silice fluorescente,
   dichlorométhane/THF (98:2),
   révélation à l'iode (Rf = 0,5)
2°) Spectre de masse : (voir tableau n° 1).

### b) Préparation de la 7-carbéthoxy-amino 4-hexyl coumarine (6c).

On introduit sous agitation 1l de mélange butanol-eau-acide sulfurique 25:5:70, 92g (0,46mol) d'heptanoyle acétate d'éthyle (**5c**) brut, 75g (0,414mol) de 3-carbéthoxyaminophénol (**2**), et on laisse sous agitation durant 24h.

On coule lentement sur 640g de glace pilée, et on laisse 1h sous agitation. On essore et on lave à l'eau le précipité. On sèche sous vide.

Rendement = 53g (40%)

Les caractéristiques du produit sont les suivantes.
1°) C.C.M.
   Silice fluorescente,
   dichlorométhane/THF (98/2),
   révélation : UV longs et courts (Rf=0,5)
2°) Spectre R.M.N. (voir tableau n° 2).

### c) Préparation de 7-amino 4-hexyl coumarine (7c).

On met en suspension 48g (0,151mol) de 7-carbéthoxy-amino 4-hexyl coumarine (**6c**) dans un mélange de 192ml d'acide sulfurique et 192ml d'acide acétique. On porte au reflux durant 5h.

On coule le mélange sur 1kg de glace et 0,5 l d'eau. En maintenant à 15°-20°C par refroidissement énergique, on ramène à pH 8 à l'aide de soude 12N. Le précipité formé est essoré et remis 3 fois en suspension dans l'eau pour éliminer tous les sels. Après séchage, le produit brut (27g) est recristallisé dans 1350ml d'éthanol.

Rendement = 18,4g (50%)

Les caractéristiques du produit sont les suivantes.
1°) C.C.M.
   Silice fluorescente,
   dichlorométhane/THF (85/5),
   révélation : UV longs et courts (Rf=0,75).
2°) Spectre RMN : (voir tableau n° 3)
3°) Spectre de masse : (voir tableau n° 3).

### Exemple 4 : Préparation de la 7-amino 4-heptyl coumarine (7d).

Pour cette préparation, on synthétisera l'octanoyl acétate d'éthyle (**5d**) en utilisant La même technique que dans l'exemple 2. On condense ce β-cétoester avec le 3-carbéthoxyaminophénol (**2**), en milieu acide, pour obtenir la 7-carbéthoxy-amino 4-heptyl coumarine (**6d**). On hydrolyse ce produit en milieu acétosulfurique pour obtenir la 7-amino 4-heptyl coumarine (**7d**).

### a) Préparation de l'octanoyle acétate d'éthyle (5d).

On introduit 285,7g (2,5mol) d'acide de Meldrum (**3**) dans 900ml de dichlorométhane anhydre. On ajoute en 1h 305g (2,5mol) de 4-diméthylamino pyridine à une température comprise entre 5° et 10°C.

On coule ensuite lentement, entre 5 et 10°C, une solution de 406,6g (2,5mol) de chlorure d'octanoyle (**4d**) dans 950ml de dichlorométhane anhydre.

Après 1h d'agitation à 5°C et 1h à 20°C, on filtre le précipité formé que l'on élimine après rinçage. Le filtrat est lavé avec 2,5 l d'acide chlorhydrique 0,6N. La phase aqueuse est extraite avec 1l de dichlorométhane. Les solutions organiques, lavées avec 1l d'eau, sont séchées puis mises à sec.

Le concentrat, repris par 4 l d'éthanol est porté au reflux durant 2h.

Après évaporation sous vide, le résidu pèse 523g (Rt=97%).

Les caractéristiques du produit sont les suivantes.
1°) C.C.M.
   Silice fluorescente,
   dichlorométhane/THF (98:2),
   révélation à l'iode (Rf=0,72)
2°) Spectre de masse : (voir tableau n°1).

### b) Préparation de la 7-carbéthoxy-amino 4-heptyl coumarine (6d).

On introduit sous agitation 245ml de mélange butanol-eau-acide sulfurique (25:5:70), 23,8g (0,1mol) d'octanoyle acétate d'éthyle (**5d**) brut, 18,2g (0,1mol) de 3-carbéthoxy amino phénol **(2)**, et on laisse sous agitation durant 24h.

On coule lentement sur 300g de glace pilée, et 375ml de dichlorométhane. Après extraction de la phase aqueuse avec 125ml de dichlorométhane et lavage des phases organiques réunies par 125ml d'eau, on sèche et on concentre à sec.

Le résidu (69g), repris par 69ml d'acétate d'éthyle, Laisse cristalliser le produit. Après séchage sous vide, on obtient 19,3g (39%) de cristaux.

Les caractéristiques du produit sont les suivantes.
1°) C.C.M.
   Silice fluorescente,
   dichlorométhane/THF (98:2),
   révélation : UV longs et courts (Rf=0,55),
2°) Spectre RMN : (voir tableau n° 2).

### c) Préparation de la 7-amino 4-heptyl coumarine (7d).

On met en suspension 5g de 7-carbéthoxy amino 4-heptyl coumarine (**6d**) dans un mélange de 20ml d'acide sulfurique et 20ml d'acide acétique. On porte au reflux durant 4 h.

On coule le mélange sur 80g de glace. On ramène à pH 8,2 à l'aide de 95ml de soude 10N. Le précipité formé est remis 2 fois en suspension dans 100ml d'eau pour éliminer les sels. Après séchage, le produit brut (4,26g) est recristallisé dans 120ml d'éthanol.

Rendement 2,59g (66%)

Les caractéristiques du produit sont les suivantes.
1°) C.C.M.
   Silice fluorescente,
   dichlorométhane/THF (98:2),
   révélation : UV longs et courts (Rf=0,4)
2°) Spectre RMN (voir tableau n° 3)
3°) Spectre de masse (voir tableau n° 3).

### Exemple 5 : Préparation de La 7-amino 4-nonyl coumarine (7é).

Pour cette préparation, on synthétisera le décanoyl acétate d'éthyle (**5e**) en utilisant la même technique que dans l'exemple 2. On condense ce β-céto ester avec le 3-carbéthoxyaminophénol (**2**) en milieu acide, pour obtenir la 7-carbéthoxy amino 4-nonyl coumarine (**6e**). On hydrolyse ce produit en milieu acéto-sulfurique pour obtenir la 7-amino 4-nonyl coumarine (**7e**).

### a) Préparation du décanoyl acétate d'éthyle (5e).

On introduit 102,7g (0,9mol) d'acide de Meldrum (**3**) dans 350ml de dichlorométhane anhydre. On ajoute en 1/2h, 110g (0,9mol) de 4-diméthylamino pyridine à une température comprise entre 5° et 10°C.

On coule ensuite, lentement, entre 5 et 10°C, une solution de 190,7g (0,9mol) de chlorure de décanoyle (**4e**) dans 330ml de dichlorométhane anhydre.

Après 1h d'agitation à 5°C et 1h à 20°C, on filtre le précipité formé que l'on élimine après rinçage. Le filtrat est lavé avec 0,8 l d'acide chlorhydrique normal. La phase aqueuse est extraite avec 2 fois 400ml de dichlorométhane. Les phases organiques sont lavées à l'eau, séchées sur sulfate de magnésium et évaporées à sec.

Le concentrat, repris par 550ml d'éthanol est porté au reflux pendant 2h. On évapore l'éthanol à sec et on utilise le produit tel quel pour le stade suivant.

Rendement = 204g (93,7%)

Les caractéristiques du produit sont Les suivantes.
1°) C.C.M.
   Silice fluorescente,
   dichlorométhane/THF (98:2),
   révélation à l'iode (Rf = 0,6)
2°) Spectre de masse : (voir tableau n° 1).

### b) Préparation de la 7-carbéthoxy amino 4-nonyl coumarine (6e).

On introduit sous agitation 1041ml d'acide sulfurique à 70%, 136,8g (0,424mol) de décanoyl acétate d'éthyle (**5e**), 77g (0,424mol) de 3-carbéthoxy amino phénol (**2**) et on laisse sous bonne agitation durant 8h.

On coule la suspension formée sur 1,2kg de glace et on ajoute, après 30 min, 1,5 l de dichlorométhane.

On sépare la phase organique et on extrait à nouveau la phase aqueuse avec 500ml de dichlorométhane. Après lavage à l'eau et séchage des phases organiques, on concentre à sec et on reprend le résidu (227g) avec 230ml d'acétate d'éthyle. Le précipité obtenu est filtré, rincé et séché sous vide.

Rendement : 13g (8,6%).

Les caractéristiques du produit sont les suivantes.
1°) C.C.M.
   Silice fluorescente,
   dichlorométhane/THF (98:2),
   révélation : UV courts et longs (Rf=0,62).
2°) Spectre RMN : (voir tableau n° 2).

### c) Préparation de la 7-amino 4-nonyl coumarine (7e).

On met en suspension 5,45g (15,2mmol) de 7-carbéthoxyamino 4-nonyl coumarine (**6e**) dans un mélange de 21,8ml d'acide sulfurique et 21,8ml d'acide acétique, et on porte au reflux durant 8h.

On coule le mélange sur 100g de glace. En maintenant à 15-20°C par refroidissement énergique, on ramène à pH 7,7 à l'aide de soude 12N (50ml). On extrait par 500ml puis 200ml de dichlorométhane et on lave les phases organiques jointes avec 200ml d'eau. Après séchage et mise à sec de la solution organique, on a un résidu de 3,6g qu'on recristallise dans 28ml d'acétate d'éthyle.

Rendement = 2,9g (66%)

Les caractéristiques du produit sont les suivantes.
1°) F=141-142°C
2°) C.C.M.
   Silice fluorescente,
   dichlorométhane/THF (98:2)
   Rf=0,35.
3°) Spectre RMN : (voir tableau n° 3)
4°) Spectre de masse : (voir tableau n° 3).

### Exemple 6 :Préparation de N-carbobenzyloxy-alanyl 4-propyl 7-coumarinylamide (9a).

On introduit 4g (19,7mmol) de 7-amino 4-propyl coumarine (**7a**), 4,4g (19,7mmol) de CBZ-L-alanine (8), 3,2g (19,7mmol) d'hydroxybenzotriazole et, à 10°C, 4,06g (19,7mmol) de dicyclohexylcarbodiimide dans 50ml de diméthylformamide.

Après 17h d'agitation à température ambiante, on filtre le précipité formé et on concentre le filtrat *à* sec. Le concentrat est repris par 40ml de dichlorométhane, qui est lavé avec 40ml de bicarbonate 0,5N, 100ml d'acide chlorhydrique 0,5N, puis séché sur sulfate de magnésium. Après mise à sec, le résidu est recristallisé dans 20ml d'acétate d'éthyle.

Rendement : 1,6g (20%)

Les caractéristiques du produit sont les suivantes.
1°) C.C.M.
Silice fluorescente KC18F,
méthanol/eau (80:20)
révélation : UV courts et longs (Rf=0,3).

Le produit contient encore un peu de 7-amino 4-propyl coumarine **(7a)**, il sera utilisé tel quel, la purification se faisant au stade suivant.

### Exemple 7 : Préparation de N-carbobenzyloxy-alanyl 4-butyl 7-coumarinylamide (9b).

On introduit 4,87g (0,0224mol) d'aminobutyl coumarine (**7b**), 5g (0,0224mol) de CBZ-L-alanine **(8)** et 4,62g (0,0224mol) de dicyclohexylcarbodiimide dans 37ml de diméthylformamide.

Après 30h d'agitation à température ambiante, on filtre Le précipité formé et on concentre le filtrat à sec. Le concentrat est repris par 70ml de dichlorométhane et 50ml de bicarbonate 1N. Il se forme un précipité qui est redissous par le diméthylformamide. Les phases organiques jointes et séchées sont mises à sec. Le résidu (11g) subit une recristallisation fractionnée dans l'acétate d'éthyle.

Rendement des fractions pures = 2,8g (29%)

Les caractéristiques du produit sont Les suivantes.
1°) C.C.M.
Silice fluorescente,
dichlorométhane/THF (85:5),
révélation : UV courts et longs (Rf=0,5).

Le produit contient encore un peu de 7-amino 4-butyl coumarine **(7b)** ; il sera utilisé tel quel, la purification se faisant au stade suivant.

### Exemple 8 : Préparation de N-carbobenzyloxy-alanyl 4-hexyl 7-coumarinylamide (9c).

On introduit 5g (20,4mmol) d'aminohexyl coumarine **(7c)**, 5,36g (24mmol) de Z-L-alanine (**8**) et 4,96 de dicyclohexyl carbodiimide, dans 37ml de diméthylformamide.

Après 24h d'agitation à température ambiante, on filtre le précipité formé puis on concentre le filtrat à sec. Le concentrat est redissous dans 130ml de dichlorométhane et cette solution lavée par 100ml de bicarbonate 1N, 100ml d'eau, est séchée et mise à sec. Le concentrat est recristallisé dans 44ml d'acétate d'éthyle.

Rendement = 3,8g (41%).

Les caractéristiques du produit sont les suivantes.
1°) C.C.M.
Silice fluorescente,
dichlorométhane/THF (85:5),
révélation : UV longs et courts (Rf=0,6).

Le produit contient encore un peu de 7-amino 4-hexyl coumarine (**7c**) ; il sera utilisé tel quel, la purification se faisant au stade suivant.

### Exemple 9 : Préparation de N-carbobenzyloxy-alanyl 4-heptyl 7-coumarinylamide (9d).

Après avoir dissous 3,45g (15,4mmol) de carbobenzyloxy L-alanine (**8**) dans 225ml de dichlorométhane anhydre, on introduit 3,55g (30,8mmol) d'éthyl morpholine et on refroidit la solution à -15°C.

On coule ensuite, à -15°C, une solution de 3,64g (15,4mmol) de chlorure de diphényl phosphinyle dans 16ml de dichlorométhane. Après 20 minutes d'agitation à -15°C, on additionne 4,41g (17mmol) de 7-amino 4-heptyl coumarine (7d) puis on agite 10min à -15°C et 24h à 20°C.

La phase organique est lavée avec 250ml d'eau, 2 fois 250ml d'acide chlorhydrique 1N, 2 fois 50ml de bicarbonate 1N et 250ml d'eau, séchée puis concentrée à sec.

Le résidu (7,73g) sera utilisé tel quel pour le stade suivant (hydrogénation).

Les caractéristiques du produit sont les suivantes.
1°) C.C.M.
Silice fluorescente,
dichlorométhane : THF (85:5),
révélation UV longs et courts (spot principal de Rf=0,3 et spot secondaire de Rf=0,5).

### Exemple 10 : Préparation de N-carbobenzyloxy-alanyl 4-nonyl 7-coumarinylamide (9e).

Après avoir dissous 3,53g (15,8mmol) de carbobenzyloxy L-alanine (**8**) dans 240ml de dichlorométhane anhydre, on introduit 3,64g (31,6mmol) d'éthyl morpholine et on refroidit la solution à -15°C. On coule ensuite à -15°C, une solution de 3,74g (15,8mmol) de chlorure de diphényl phosphinyle dans 16ml de dichlorométhane. Après 20min d'agitation à -15°C, on additionne 5g (17,4mmol) d'aminononyl coumarine (**7e**) puis on agite 10min à -15°C et 24h à 20°C.

La phase organique est lavée avec 250ml d'eau, 2 fois 250ml d'acide chlorhydrique 1N, 2 fois 50ml de bicarbonate 1N et 250ml d'eau, séchée puis concentrée à sec.

Le résidu est chromatographié sur colonne de silice avec comme éluant du dichlorométhane - THF (85:5). Les fractions utilisables pour le stade suivant représentent 5g (64%).

Les caractéristiques du produit sont les suivantes.
1°) C.C.M.
Silice fluorescente,
dichlorométhane/THF (85:5),
révélation : UV longs et courts (spot principal de Rf = 0,4 et spot secondaire de Rf=0,55).

Le produit contient encore un peu de 7-amino 4-nonylcoumarine ; il sera utilisé tel quel, la purification se faisant au stade suivant.

### Exemple 11 : Préparation de L-alanyl 4-propyl 7-coumarinylamide, chlorhydrate (10a).

On dissout 1,6g (4mmol) de N-carbobenzyloxy-L-alanyl 4-propyl 7-coumarinylamide (**9a**) dans 50ml de tétrahydrofuranne. Après introduction de 320mg de palladium sur charbon à 10%, on hydrogène sous bonne agitation durant 5h.

Après filtration du catalyseur, le filtrat est concentré à sec et le produit chromatographié sur colonne de silice, avec comme éluant du dichlorométhane méthanol (80:20).

Les fractions de chromatographie sont concentrées à sec puis dissoutes dans 7ml de tétrahydrofuranne. On salifie avec une solution éthanolique d'acide chlorhydrique.

Rendement = 390mg (36%)

Les caractéristiques du produit sont les suivantes.
1°) Point de fusion : 227-228°C
2°) C.C.M.
   Silice fluorescente,
   dichlorométhane : méthanol (80:20),
   révélation : UV longs et courts/ninhydrine, Rf=0,3
3°) Spectre R.M.N. (voir tableau n° 4)
4°) Spectre de masse (voir tableau n° 4)

### Exemple 12 : Préparation de L-alanyl 4-butyl 7-coumarinylamide, chlorhydrate (10b).

On dissout 2,5g (0,0059mol) de N-carbobenzyloxy alanine 4-butyl 7-coumarinylamide (**9b**) dans 75ml de tétrahydrofuranne. Après introduction de 500mg de palladium sur charbon à 10%, on hydrogène sous bonne agitation durant 5h.

Après filtration du catalyseur, le filtrat est concentré à sec et le produit chromatographié sur colonne de silice avec comme éluant du dichlorométhane-méthanol (85:15).

Les fractions pures sont concentrées à sec puis dissoutes dans 17ml de tétrahydrofuranne. On salifie avec une solution éthanolique d'acide chlorhydrique. Le produit est rincé à l'éther.

Rendement = 0,8g (42%)

Les caractéristiques du produit sont les suivantes.
1°) C.C.M.
   Silice fluorescente,
   dichlorométhane/méthanol (85:15),
   révélation : UV longs et courts/ninhydrine (Rf=0,4).
2°) Spectre R.M.N. : (voirtableau n° 4)
3°) Spectre de masse : (voir tableau n° 4).

### Exemple 13 : Préparation de L-alanyl 4-hexyl 7-coumarinylamide, chlorhydrate (10c).

On dissout 3,8g de N-carbobenzyloxy alanine 4-hexyl 7-coumarinylamide (**9c**) dans 100ml de tétrahydrofuranne. Après introduction de 760mg de palladium sur charbon à 10%, on hydrogène sous bonne agitation durant 16h.

Après filtration du catalyseur, le filtrat est évaporé à sec et repris dans 50ml de tétrahydrofuranne. On salifie avec une solution éthanolique d'acide chlorhydrique. Après addition de 50ml d'éther et refroidissement, on filtre et rince à l'éther.

Rendement = 1,4g (47%).

Les caractéristiques du produit obtenu sont les suivantes.
1°) C.C.M.
   Silice fluorescente,
   dichlorométhane/éthanol (80/20),
   révélation : UV longs et courts/ninhydrine (Rf=0,48)
2°) Spectre R.M.N. : (voir tableau n° 4)
3°) Spectre de masse : (voir tableau n° 4).

### Exemple 14 : Préparation de L-alanyl 4-heptyl 7-coumarinylamide, chlorhydrate (10 d)

On dissout 7,73g (16,6mmol) de N-carbobenzyloxy L-alanyl 4-heptyl 7-coumarinylamide (**9d**) dans 231ml de tétrahydrofuranne. Après introduction de 1,54g de palladium sur charbon à 10%, on hydrogène sous bonne agitation durant 5 heures 30.

Après filtration du catalyseur, le filtrat est concentré à sec et le produit chromatographié sur colonne de silice avec comme éluant du dichlorométhane : méthanol (9:1, puis 7:3).

Les fractions de chromatographie sont concentrées à sec (obtention de 0,9g) puis dissoutes dans 15ml de tétrahydrofuranne. On salifie avec une solution éthanolique d'acide chlorhydrique.

Rendement : 0,89g (16%)

Les caractéristiques du produit sont les suivantes :
1°) C.C.M.
   silice fluorescente
   butanol : acide acétique : eau (60:20:20),
   révélation : UV courts et longs / ninhydrine (Rf=0,7)
2°) Spectre RMN (tableau n° 4)
3°) Spectre de masse (tableau n° 4).

### Exemple 15 : Préparation de L-alanyl 4-nonyl 7-coumarinylamide, chlorhydrate (10 e)

On dissout 5g (10,1mmol) de N-carbobenzyloxy L-alanyl 4-nonyl 7-coumarinylamide **(9e)** dans 150ml de tétrahydrofuranne. Après introduction de 1g de palladium sur charbon à 10%, on hydrogène sous bonne agitation durant 8 heures.

Après filtration du catalyseur, le filtrat est concentré à sec et le produit chromatographié sur colonne de silice avec comme éluant les mélanges dichlorométhane : éthanol (9:1, puis 7:3).

Les fractions de chromatographie sont concentrées à sec (obtention de 1,76g) puis dissoutes dans 28ml de tétrahydrofuranne. On salifie avec 1,08ml d'une solution méthanolique d'acide chlorhydrique 5,1N, puis ajout de 200ml d'éther.

Rendement = 1,6g (39,8%)

Les caractéristiques du produit sont les suivantes.
1°) C.C.M.
   silice fluorescente
   butanol : acide acétique : eau (60:20:20),
   révélation : UV courts et longs / ninhydrine (Rf = 0,75)
2°) Spectre RMN (tableau n° 4)
3°) Spectre de masse (tableau n° 4).

### Exemple 16 : Préparation du N-carbo-t-butyloxy N-ω-nitro arginyl-4- hexyl coumarinylamide de formule :

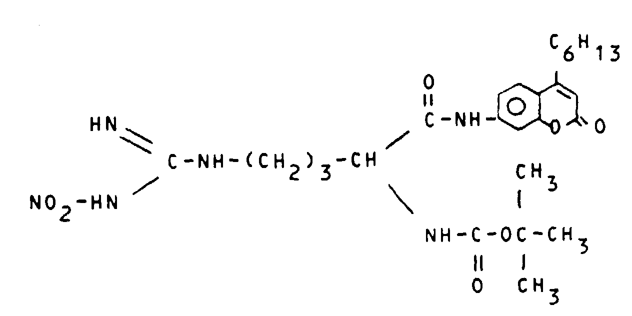

Après avoir dissous 7,77g (24,3 mmol) de N-carbo-t-butyloxy N- ω nitro arginine dans 116ml de diméthylformamide, on introduit 5,61g (48,7 mmol) d'éthyl morpholine et on refroidit la solution à -12°C.

On coule ensuite, vers -15°C, une solution de 5,76g (24,3mmol) de chlorure de diphényl phosphinyle dans 24ml de diméthylformamide. Après 20 minutes d'agitation à -15°C, on additionne 4,78g (19,5mmol) de 7-amino 4-hexyl coumarine (**7 c**) puis on agite 10 minutes à -15°C et 48 heures à température ambiante.

L'insoluble est filtré et rincé au diméthylformamide, et le filtrat est mis à sec sous vide. Le concentrat est repris par 195ml de dichlorométhane / acétate déthyle (1:1). Après élimination d'un insoluble d'amino hexyl coumarine, on lave la solution au bicarbonate 1N, puis à l'eau et on met à sec. Le résidu, repris dans 40ml de dichlorométhane, puis filtré, est chromatographié sur colonne de silice avec le dichlorométhane-éthanol (93:7) comme éluant.

Les fractions purifiées représentent 600mg (9%).

Les caractéristiques du produit sont les suivantes.
1°) C.C.M.
   Silice fluorescente
   dichlorométhane : éthanol (93:7),
   révélation : UV longs et courts (Rf=0,32)
2°) RMN (CDCl₃) : 0,88 (t, 3H, CH₃-CH₂), 1,39 (m, 21 H, t Bu / (CH₂)₂ arg / (CH₂)₄ coum) ; 2,70 (t, 2H, CH₂- 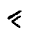 ) ; 3,39 (t, 2H, CH₂NH) 4,59 (t, 1H, *CH ) ; 5,88 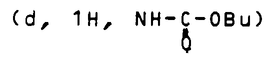 6,16 (5,1H, = CH) ; 7,53 (m,4H, Harom/NH) ; 8,65 (m, 1H, NH); 9,66 (5,1H, NH)

### Exemple 17 : Préparation du N-ω-nitro argininyl 4-hexyl coumarinylamide, trifluoroacétate de formule :

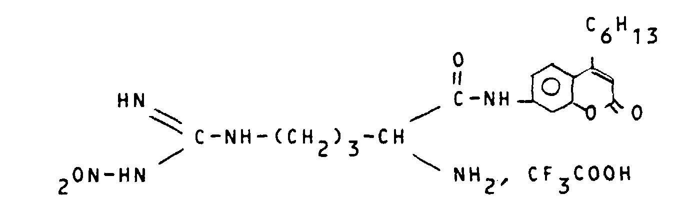

Pour cette préparation, on réalise l'acidolyse dans l'acide trifluoracétique du N-carbo t-butyloxy N-ω-nitro arginyl coumarinylamide.

On met en solution 1,4g (2,56mmol) de N-carbo t-butyloxy N-ω-nitro arginyl coumarinylamide dans 22ml d'acide trifluoracétique.

Après 1h de contact sous agitation, on évapore à sec et on reprend le résidu dans 67ml d'éther. Les cristaux obtenus, filtrés, sont bien rincés à l'éther et séchés sous vide.
Rt = 1,14g (80%).

RMN (CDCl₃ - CD₃OD) : 0,86 (t, 3H, CH₃-CH₂) ; 1,14 (m, 6H, CH₂ x 3) ; 1,64 (m, 6H, CH₂ - CH₂ - *CH / 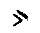 - CH₂ - CH₂) ; 2,53 (t, 2H, CH₂ - 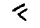 ) ; 3,09 (t, 2H, CH₂NH) ; 3,90 (t, 1H, *CH) ; 5,97 (s, 1H, = CH) ; 7,21 (dd, 1H, Har) ; 7,39 (d, 1H, Harom) 7,53 (d, 1H, Har).

### Exemple 18 : N-carbo t-butyloxy valyl - prolyl - N-ω-nitroarginyl 4-hexyl coumarinylamide de formule :

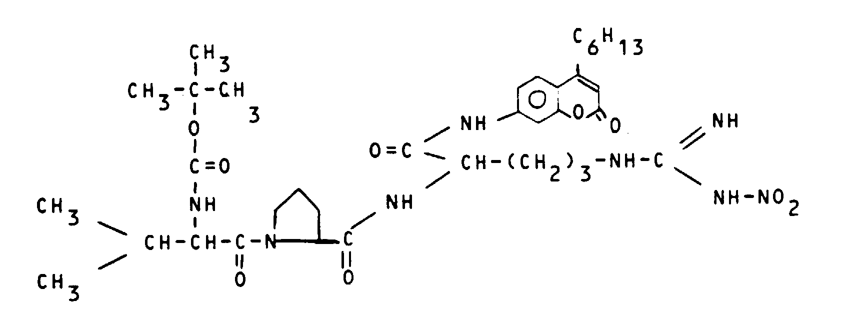

Pour cette synthèse, on prépare un anhydride mixte du N-carbo t-butyloxy valyl proline (t-Boc Val-Pro (OH)) par action du chlorure de diphényl phosphinyle. Cet anhydride mixte est ensuite couplé au N-ω nitroarginyl 4-hexyl coumarinylamide.

Après avoir dissous 0,674g (2,14mmol) de t-Boc-valine-proline dans 15ml de dichlorométhane, on introduit 0,494g (4,29mmol) d'éthyl morpholine et on refroidit la solution à -16°C. On coule ensuite à la même température 0,508g (2,14mmol) de chlorure de diphényl phosphinyle dissous dans 1ml de dichlorométhane, et on laisse en contact 20 minutes à la même température.

Pendant ce temps, on désalifie une solution de 1,173g (1,95mmol) de N-ω-nitro arginyl 4-hexyl coumarinylamide, 1,24 trifluoroacétate, dissous dans 6,45ml de dichlorométhane, avec 307mg d'éthyl morpholine puis on coule cette solution sur la solution précédente. On laisse 10 minutes à -15°C puis 24 heures à température ambiante.

Après lavage du milieu réactionnel à l'eau, à l'acide citrique 1N, au bicarbonate 1N, puis à l'eau, on sèche et on évapore à sec.

Résidu = 1,15 g.

Les caractéristiques du produit sont les suivantes.
1°) C.C.M.
   Silice fluorescente
   dichlorométhane : éthanol (93:7),
   révélation : UV longs et courts / vert de bromocrésol / permanganate basique (Rf = 0,35).
2°) RMN (CDCl₃ - CD₃OD) : 0,90 (m, 9H, CH₃-CH₂/CH₃-CH-CH₃ ; 1,30 (m, 15H, tBu/(CH₂)₃) ; 1,90 (m, 11H CH₃-CH-CH₃ / CH₂-CH₂-*CH ≥ -CH₂-CH₂ 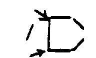 N) ; 2,71 (t, 2H, CH₂- 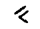 ) ; 3,21 (t, 2H, CH₂N) ; 3,60 (m, 2H, CH₂N pro) ; 4,14 (d, 1H, α CHVal) ; 4,51 (m, 2H, C*Hpro + C*Harg) ; 6,15 (s, 1H, CH =) ; 7,48 (m, 3H, Harom) ; 7,68 (m, 2H, 2 x NH) ; 7,90 (d, 1H, NH) ; 10,00 (s,1H, NH).

### Exemple 19 : N-carbo t-butyloxy valyl-prolyl arginyl 4-hexyl coumarinylamide, acétate de formule :

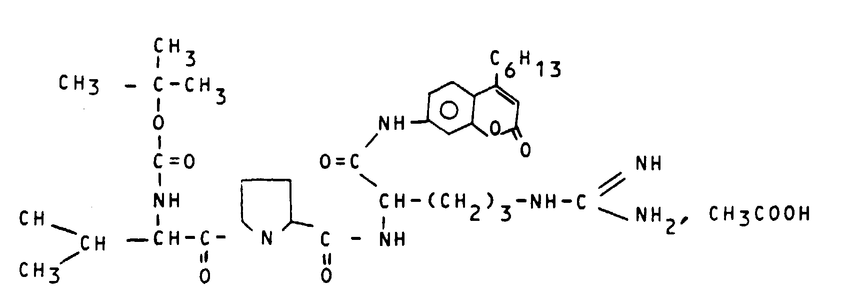

Pour cette synthèse, on réalise la déprotection du groupement ω-nitro arginyl par hydrogénolyse en milieu hydro-acétique.

1,1g (1,48mmol) de N-carbo t-butyloxy valyl-prolyl arginyl 4-hexylcoumarinylamide sont mis en suspension dans 17ml d'un mélange d'acide acétique-eau (60/40) et hydrogénés en présence de 132mg de palladium sur charbon à 10%. L'hydrogénation dure 14 heures avec deux nouveaux ajouts successifs de 66 et 132mg de palladium.

Après filtration du catalyseur et rinçage à l'eau et au méthanol, on évapore le solvant sous vide. Le résidu est repris par le dichlorométhane, la solution filtrée et le filtrat déposé sur une colonne de silice montée sur dichlorométhane. Le produit est chromatographié par élution au dichlorométhane-éthanol-acide acétique (70:15:15). Les fractions de produit purifié sont mises à sec puis reprises dans 50ml d'éther pour la cristallisation.

Rendement = 360mg (33%).

Les caractéristiques du produit sont les suivantes.
1°) C.C.M.
   Silice fluorescente
   butanol : acide acétique : eau 60/20/20,
   révélation : UV courts et longs / vert de bromocrésol / ninhydrine (Rf = 0,7, spot mauve).
2°) RMN (DMSOD₆) : 0,87 (m, 9H, CH₃-CH₂ / CH₃-CH-CH₃) ; 1,30 (m, 15H, tBu / (CH₂)₃) ; 1,74 (m, 14H, CH₃-CH-CH₃ / CH₂-CH₂-*CH C / 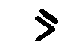 -CH₂-CH₂ / 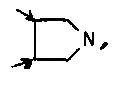 CH₃(COOH); 2,30 - 2,89 (m, 4H, CH₂Narg / CH₂- 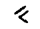 ) ; 3,08 - 4,39 (m, 5H, CH₂N pro / α CH Val / *CH pro / C*Harg) ; 6,20 (s, 1H, = CH) ; 6,84 - 7,95 (m, 8H, Harom/ NH₃⁺ / NH) ; 8,54 (d, 1H, NH) ; 8,95 (d, 1H, NH) ; 10,8 (s, 1H, NH).

### Exemple 20 : Préparation du N-carbo benzyloxy arginyl 4-hexylcoumarinylamide, chlorhydrate de formule :

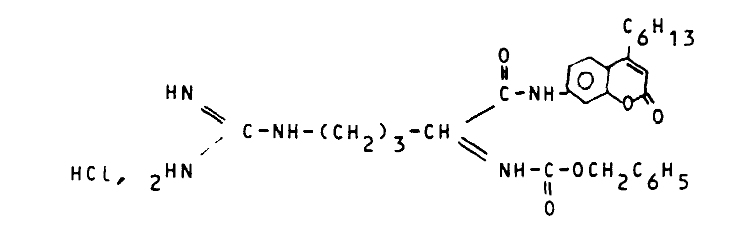

On introduit sous agitation, dans 300ml de diméthylformamide, 15g (61,2mmol) de 7-amino 4-hexyl coumarine, 42,18g (122,4mmol) de carbobenzyloxy arginine chlorhydrate, puis 12,6g (61,2mmol) de dicyclohexyl carbodiimide.

Après 22 heures d'agitation, on filtre la dicyclohexylurée formée, on lave avec 25ml de diméthylformamide et on évapore sous vide.

Le résidu (88g) est repris par 40ml de méthanol et 400ml d'acétate d'éthyle. L'insoluble est filtré, lavé avec 3 fois 50ml d'acétate d'éthyle puis séché. Le solide séché est repris par 18ml de diméthylformamide et 60ml de méthanol ; après élimination d'un insoluble, le filtrat est dilué avec 240ml d'acétate d'éthyle.

Le précipité formé est Lavé à l'acétate d'éthyle, séché puis repris sous agitation, durant 2 heures dans 10 volumes d'eau, filtré, lavé à l'eau et séché.

Rendement = 11,5g (33%).

Les caractéristiques du produit sont les suivantes.
1°) F = 142°C.
2°) C.C.M.
   silice fluorescente
   butanol / acide acétique / eau (60/20/20),
   révélation : UV longs et courts / vert de bromocrésol (Rf = 0,7).
3°) RMN (DMSOD6) : 0,84 (t,3H, CH₃-CH₂) ; 1,43 (m, 12H, CH₂) ; 2,50 (t, 2H, CH₂-C= ) ; 2,72 (t,2H, CH₂NH) ; 3,14 (t, 1H, CH) ; 5,04 (s, 2H, O-CH₂ φ ) ; 6,20 (s, 1H, = CH) ; 7,6 (m, 8H, Harom) ; 7,98 (s, 1H, NH) ; 10,8 (s, 1H, NH).

### Exemple 21 : Préparation de l'arginyl 4-hexyl coumarinylamide, chlorhydrate, acétate de formule :

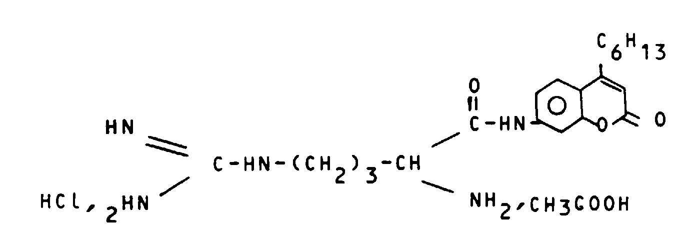

Ce produit est préparé par hydrogénation catalytique du N-carbobenzyloxy arginyl 4-hexylcoumarinylamide chlorhydrate.

On dissout 910mg de N-carbobenzyloxy arginyl 4-hexyl coumarinylamide dans un mélange de 157ml de méthanol, 31,4ml d'eau, et 15,7ml d'acide acétique. Après introduction de 91mg de palladium sur charbon à 10%, on hydrogène sous bonne agitation durant 6 heures.

Après filtration du catalyseur, le filtrat est concentré à sec et le résidu repris dans 100ml d'éther.

Rendement : 545mg ; produit hygroscopique.

Les caractéristiques du produit sont les suivantes.
1°) C.C.M.
   Silice fluorescente
   butanol : acide acétique : eau (60/20/20),
   révélation : UV longs et courts / ninhydrine / vert de bromocrésol (Rf = 0,5).
2°) RMN (CD₃OD) : 0,90 (t, 3H, CH₃-CH₂) ; 1,36 (m, 6H, CH₂ x 3) ; 1,67 (m, 6H, CH₂ - CH₂ - *CH / 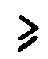 -CH₂ - CH₂) ; 1,93 (s, 3H, CH₃COOH) ; 2,78 (t, 2H, CH₂- 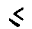 ) ; 3,29 (t, 2H, CH₂NH) ; 3,98 (t, 1H, *CH) ; 6,19 (s, 1H, = CH ) ; 7,72 (m, 3H, Har).

**Exemple 22.** Cet exemple illustre l'utilisation des dérivés de coumarines des exemples 13, 14 et 15 pour La différenciation des bactéries Gram(+) et Gram(-)par incorporation de ces dérivés de coumarines et révélation des colonies fluorescentes.

Cette différenciation fait appel à une méthode spécifique de discrimination optique des bactéries à coloration de Gram(+) ou Gram(-), qui est réalisée par l'utilisation de substrats fluorogénes de la L-alanine amino arylpeptidase, activité enzymatique retrouvée principalement dans la paroi des bactéries à coloration de Gram(-), comme il est décrit par Cerny G dans European Journal of Applied Microbiology, 1976, 3, p. 223-225, et 1978 5, p. 113-122.

La coloration de Gram est la première étape habituelle de différenciation des bactéries avant d'autres manipulations conduisant à l'identification des micro-organismes.

Une méthode alternative comme le test à la potasse décrit par Halebian S dans Journal of Clinical Microbiology, 1981, 13, p. 444-448, est fondée sur la lyse, avec relargage de macrofragments d'ADN, des bactéries Gram(-) exposées à une solution à 3% de KOH, phénomène mis en évidence par l'augmentation de la viscosité de la suspension bactérienne Gram négative. Ce test KOH est simple et rapide ; il existe cependant une mauvaise corrélation avec la technique de coloration de Gram.

Une autre méthode permet de détecter spécifiquement et rapidement certaines activités enzymatiques bactériennes, retrouvées essentiellement et quantitativement chez les bactéries à coloration de Gram(-), grâce à l'utilisation de substrats fluorogènes. La sensibilité et la rapidité de la méthode est telle qu'elle est réalisable sans isolement préalable comme il est décrit par Manafi M dans Microbiological Review, 1991, 55(3), p. 335-348. Les réactions enzymatiques sont testées dans leur globalité ou individuellement, que ce soit en milieu liquide ou en milieu solide, mais cette méthode ne peut s'appliquer aux substrats qui diffusent dans la gélose. De plus, la méthode d'incorporation dans le milieu de substrats fluorogènes ne doit pas provoquer d'inhibition du développement bactérien. En particulier, il est intéressant de n'employer que de faibles concentrations de produits purs dans une gamme maximale de 50 à 100µg/ml, tout en conservant une réaction franche et une méthode reproductible.

Dans cet exemple, on utilise cette méthode de détection des activités enzymatiques bactériennes de la L-alanine aminoaryl peptidase avec les substrats fluorogènes S107, S108 et S109 utilisant les composés 10e, 10c et 10d des exemples 15, 13 et 14. Ces substrats sont incorporés à la gélose selon la méthode énoncée par Manafi & Kneifel dans Journal of Applied Bacteriology 1990, 69, p. 822-827.

Tous les substrats (composés 10c, 10d et 10d) sont incorporés à la gélose à différentes concentrations couvrant une gamme de 10 à 300µg/ml.

Les composés sont préalablement dissous dans le DMSO et dilués dans de l'eau bi-distillée pour constituer des solutions mères à 800µg/ml, qui peuvent être conservées au moins une semaine à +4°C. Les solutions mères sont diluées ensuite pour délivrer les paliers de concentrations définis à 10, 50, 100, 200 et 300µg/ml.

Ces substrats sont ajoutés à la gélose à 45°C (une température de 85°C fut tolérée par les substrats), préalablement tamponnée à pH=7 et stérilisée par autoclavage. Les boîtes de gélose sont ensuite ensemencées d'une suspension de colonies, stérilement, à l'öse de platine par stries transversales et selon la méthode des quadrants. L'incubation est poursuivie durant une nuit à 37°C.

Pour chaque concentration on réalise un banc sans colonies et pour chaque type bactérien, un contrôle de culture sans substrat, de sorte que l'on ne puisse observer que les effets de la présence simultanée des colonies et du substrat incorporé à la gélose.

La révélation des bactéries Gram(-), rendues fluorescentes par le fluorophore S107, S108 ou S109 libéré est réalisée sous lampe UV à une longueur d'onde d'excitation de 365nm, compatible avec les dispositifs commercialisés. L'émission fluorescente impressionne un papier film 35mm Kodak® Ektar® 1000/30° pour un temps d'exposition au 1/60ème de seconde-process C-41.

Après incubation, la lecture des boites de gélose donne les résultats reportés dans le tableau 5 annexé. Les substrats S108 et S109 s'avèrent très efficaces pour réaliser la discrimination des bactéries Gram négatives des bactéries Gram positives en accord avec la technique de coloration de Gram.

Les colorants ne diffusent pas dans la gélose, de sorte que la fluorescence n'est émise que par les colonies bactériennes au site de leur croissance. Egalement, les réactions positives (fluorescence claire, lumineuse intense, des colonies Gram négatives sur un fond translucide, estompé bleu/vert pâle, fluorescence d'intensité plus faible pour les bactéries Gram positives) s'observent dès les concentrations faibles à 10µg/ml, ce qui confirme l'utilité de la nature hydrophobe des substrats, de l'absence de diffusion qui en découle et donc de l'efficacité des substrats préparés.

Leur toxicité vis-à-vis des bactéries s'avère nulle pour les substrats S108 et S109 ou faible pour le S107. Cette faible toxicité serait à corréler à une forte concentration (300µg/ml), et/ou à la longueur de la chaîne alkyle substituant en 4 (S107). Ces résultats concernant le substrat S107 suggèrent une substitution en 4 plus adéquate, et l'utilité d'un compromis diffusion/toxicité.

On observe une cinétique de l'émission fluorescente, avec une distinction dans la capacité d'hydrolyser les substrats, très importante pour les Gram(-). L'émission de fluorescence, de la sorte, peut être accumulée par une observation dans le temps, une cinétique enzymatique, ou par tout autre technique récente d'acquisition de données afin que la précision des mesures permette la définition d'une véritable "empreinte", éléments de spécificité révélateurs des espèces bactériennes étudiées.

Les résultats du tableau 5 montrent ainsi que, comme complément à la coloration Gram traditionnelle, le test amino arylpeptidase permet une différenciation de Gram plus juste, plus sensible et plus spécifique.

Ainsi, les substrats de l'invention tels que les substrats S107, S108 et S109, présentent de nombreux avantages. En effet, ils sont non diffusibles et non toxiques, ils peuvent être utilisés à très faibles concentrations (10µg/ml), permettent une interprétation simple et spécifique des résultats et conviennent à une mise en oeuvre rapide avec des temps d'incubation courts. Ils sont donc bien adaptés à la réalisation de contrôles de routine.

**TABLEAU 1**

| **Analyse spectroscopique des alcanoyl acétates d'éthyle** | | |
|---|---|---|
| Produit (masse moléculaire) | Spectre de masse (m/Z) ionisation à l'ammoniac | |
| Valeroyl acétate d'éthyle C₉H₁₆O₃ M=172,22 | 190 (M⁺ + 17) 173 (M⁺) | |
| Heptanoyl acétate d'éthyle C₁₁H₂₀O₃ M=200,3 | 218 (M⁺ + 17) 201 (M⁺) | |
| Octanoyl acétate d'éthyle C₁₂H₂₂O₃ M=214,3 | 232 (M⁺ + 17) 215 (M⁺ | |
| Décanoyl acétate d'éthyle C₁₄H₂₆O₃ M=242,35 | 272 (M⁺ + 17) 243 (M⁺) | |

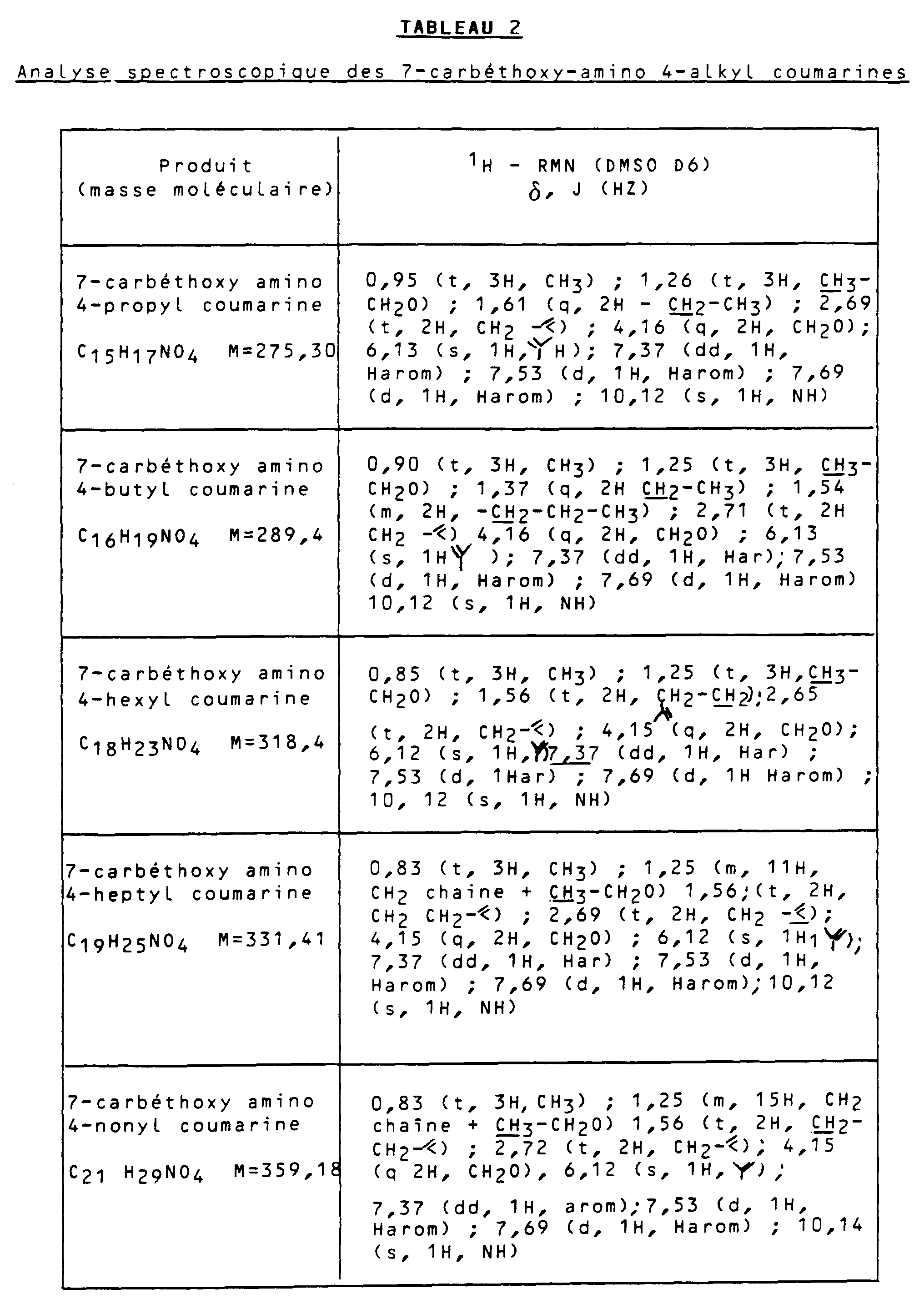

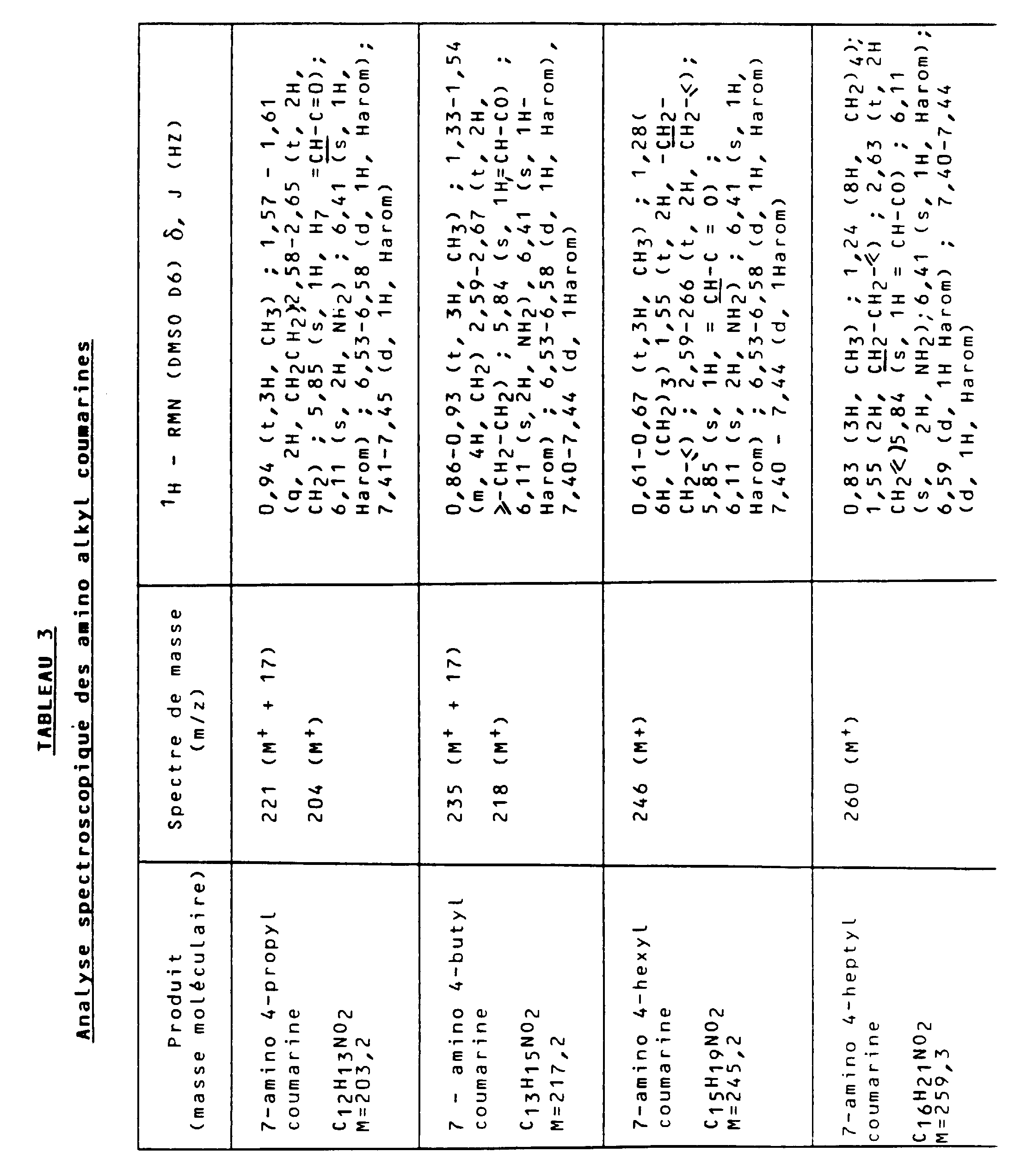

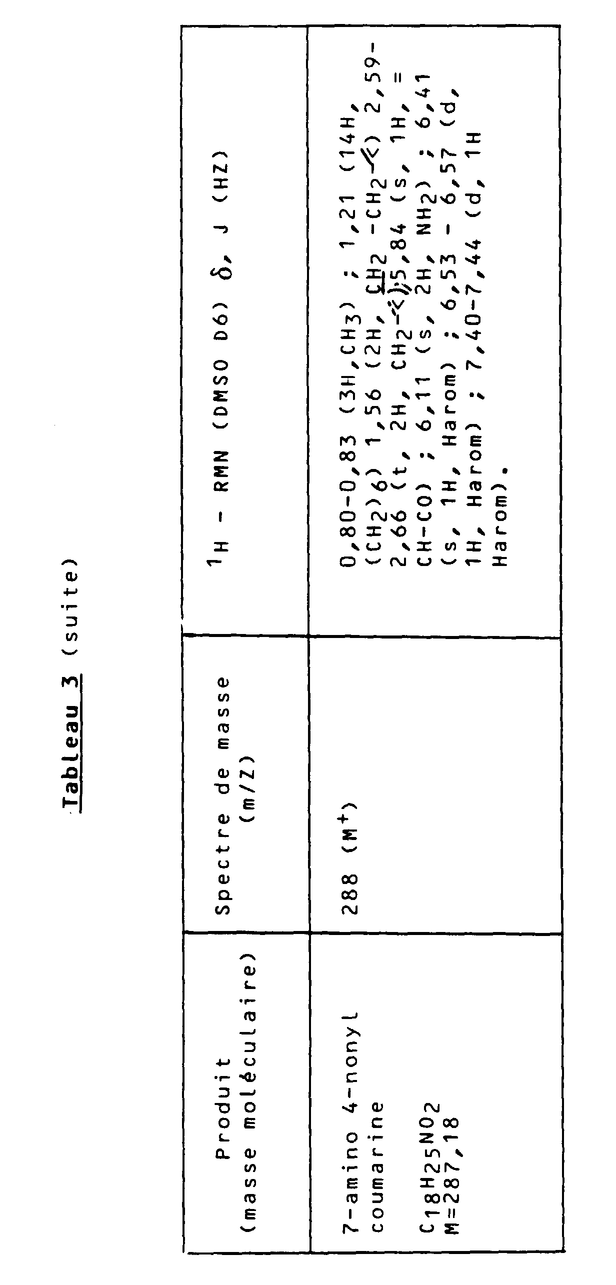

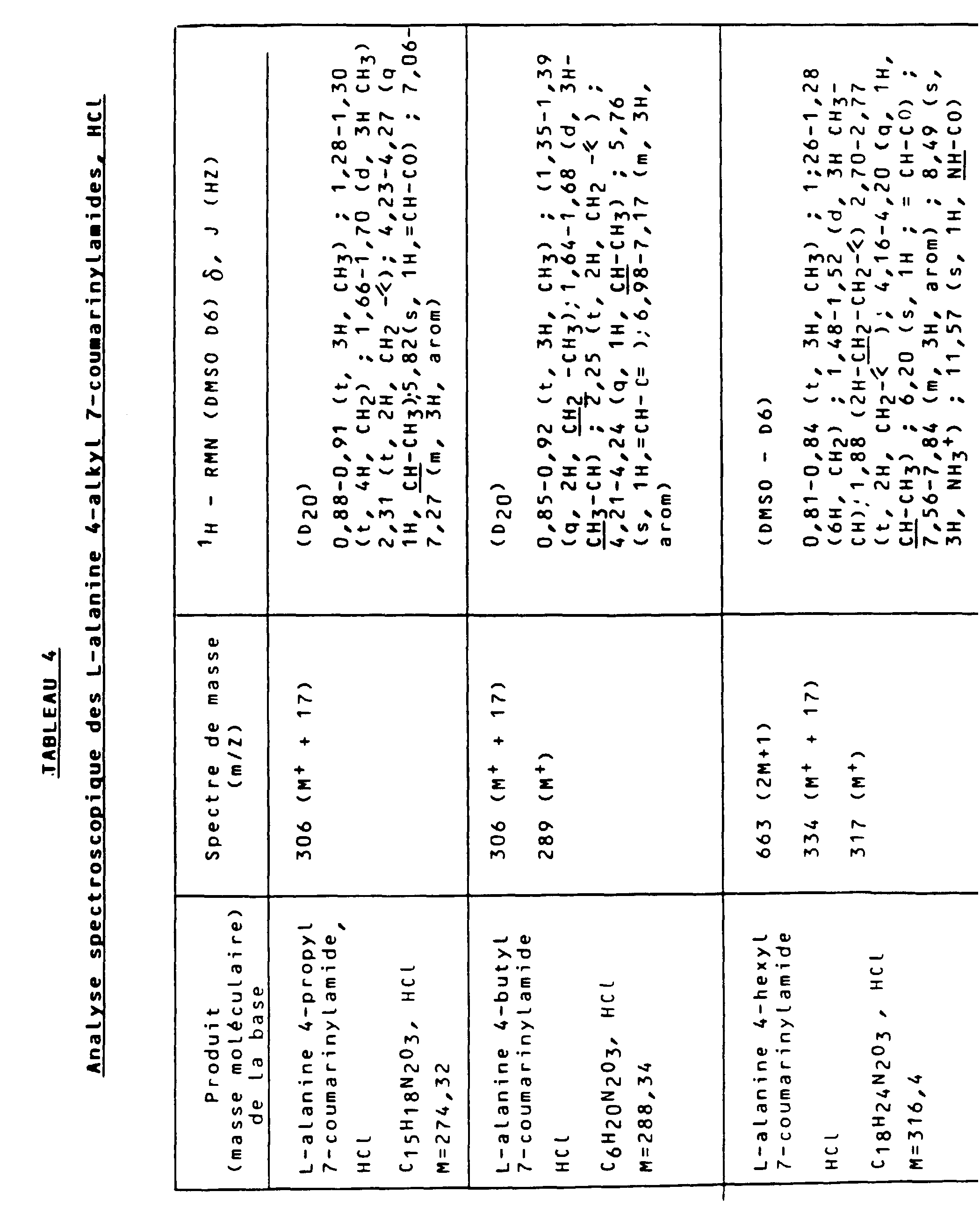

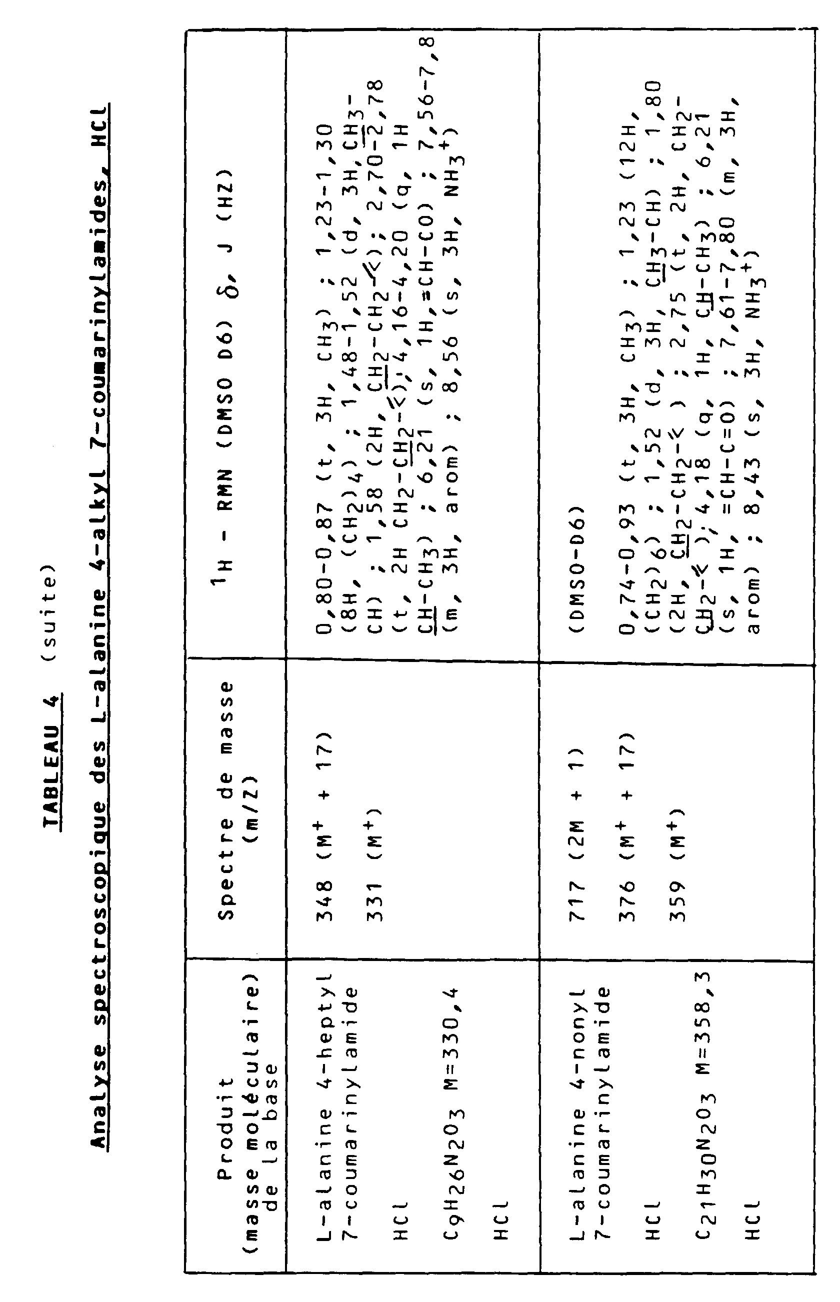

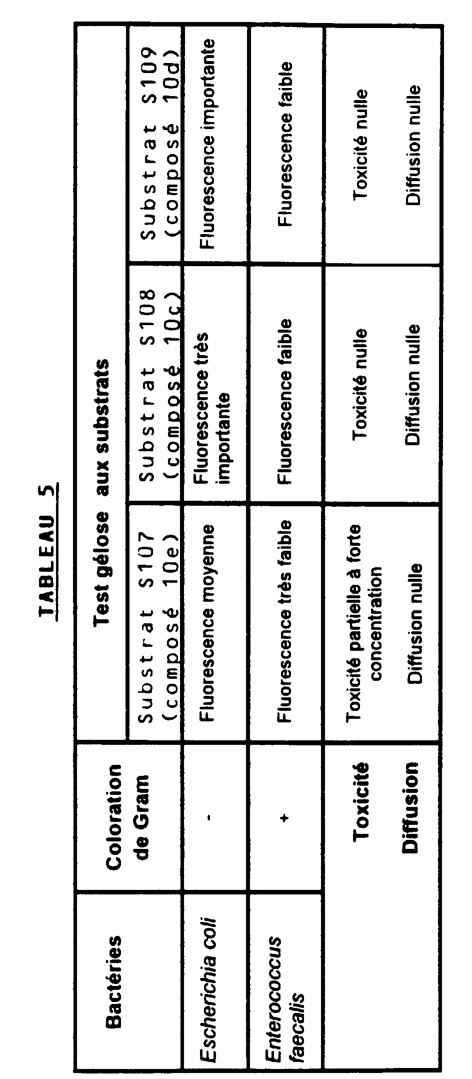

## Revendications

1. Dérivé d'aminocoumarine répondant à la formule : 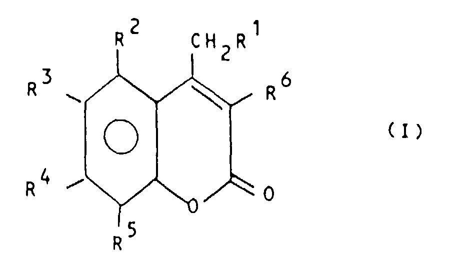 dans laquelle
1°) R¹ représente un groupe choisi parmi les groupes alkyle, linéaires ou ramifiés, en C₄ à C₁₇, les groupes cycloalkyle en C₃ à C₁₀ et les groupes aryle en C₆ à C₁₄, le groupe alkyle, cycloalkyle ou aryle étant non substitué ou substitué par au moins
- un atome d'halogène,
2°) l'un des R², R³, R⁴ et R⁵ représente
- NH₂
- NHR⁷
- NH-COOR⁷
- N(R⁸)COOR⁷
- NHR⁹, ou
- NR⁷R⁹
avec R⁷ et R⁸ identiques ou différents, représentant un groupe alkyle en C₁ à C₁₇, ou un groupe aryle en C₆ à C₁₄, ou avec R⁷ et R⁸ formant ensemble un cycle hydrocarboné, saturé ou insaturé, comportant éventuellement un hétéroatome choisi parmi O, S, et N, et R⁹ représentant un groupe acyle, dérivé d'un composé choisi parmi Les acides aminés, les peptides, les acides carboxyliques et les acides gras, le groupe amine de l'acide aminé ou du peptide étant protégé ou non protégé, salifié ou non salifié ;
3°) les autres R², R³, R⁴ et R⁵ qui peuvent être identiques ou différents, représentent
- un atome d'hydrogène, ou
- un groupe alkyle ou alcoxy, non substitué ou substitué par au moins un atome d'halogène, ou
R² et R³, R³ et R⁴ ou R⁴ et R⁵ forment ensemble un cycle hydrocarboné saturé ou insaturé de 5 à 8 atomes de carbone, ou
R³, R⁴ et R⁵, ou R², R³ et R⁴ forment ensemble avec le noyau phényle auquel ils sont liés un noyau polycyclique à 3 cycles condensés ; et
4°) R⁶ représente
- un atome d'hydrogène, ou
- un groupe choisi parmi les groupes alkyle en C₁ à C₁₇, aryle en C₆ à C₁₄ et cycloalkyle en C₃ à C₁₀, le groupe alkyle, aryle ou cycloalkyle étant non substitué ou substitué par au moins un atome d'halogène, et ses sels d'addition à des acides.

2. Dérivé selon la revendication 1, caractérisé en ce que R¹ représente un groupe alkyle de 4 à 8 atomes de carbone.

3. Dérivé selon la revendication 1, caractérisé en ce que R¹ représente le groupe butyle ou pentyle.

4. Dérivé selon l'une quelconque des revendications 1 à 3, caractérisé en ce que R⁴ représente NH₂, NHR⁷, NHCOOR⁷, NHR⁹ ou NR⁷R⁹.

5. Dérivé selon l'une quelconque des revendications 1 à 3, caractérisé en ce que R⁴ représente NH₂, NHCOOR⁷ ou NHR⁷.

6. Dérivé selon l'une quelconque des revendications 1 à 3, caractérisé en ce que R⁴ représente NHCOOR⁷ avec R⁷ représentant le groupe éthyle.

7. Dérivé selon l'une quelconque des revendications 1 à 3, caractérisé en ce que R⁴ représente NHR⁹ ou NR⁷R⁹.

8. Dérivé selon l'une quelconque des revendications 1 à 3, caractérisé en ce que R⁴ représente NHR⁹ avec R⁹ représentant le groupe acyle dérivé de l'alanine protégé par le groupe benzyloxycarbonyle ou salifié par l'acide chlorhydrique.

9. Dérivé selon l'une quelconque des revendications 1 à 3, caractérisé en ce que R⁴ représente NHR⁹ avec R⁹ représentant le groupe acyle dérivé de l'arginine ou de l'arginine protégée par le groupe t-butyloxycarbonyle ou benzyloxycarbonyle, ou salifié par HCl ou CH₃COOH.

10. Dérivé selon l'une quelconque des revendications 1 à 3, caractérisé en ce que R⁴ représente NHR⁹ avec R⁹ représentant le groupe acyle dérivé du tripeptide -Arg-Pro-Val protégé par le groupe t-butoxycarbonyle.

11. Dérivé selon l'une quelconque des revendications 4 à 10, caractérisé en ce que R², R³, R⁵ et R⁶ représentent un atome d'hydrogène.

12. Utilisation d'un dérivé d'aminocoumarine de formule : 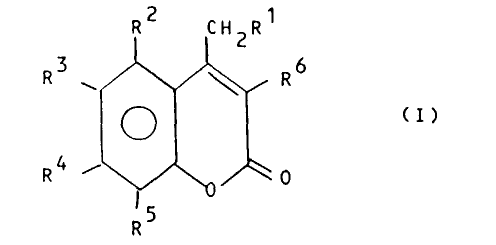 dans laquelle
1°) R¹ et R⁶ sont tels que définis dans la revendication 1,
2°) l'un des R², R³, R⁴ et R⁵ représente -NH₂, -NHR⁷ ou -NHCOOR⁷ avec R⁷ ayant la signification donnée dans la revendication 1, et les autres R², R³, R⁴ et R⁵ qui peuvent être identiques ou différents, représentent
- un atome d'hydrogène, ou
- un groupe alkyle ou alcoxy, non substitué ou substitué par au moins un substituant choisi parmi les atomes d'halogène, ou
R² et R³, R³ et R⁴ ou R⁴ et R⁵ forment ensemble un cycle hydrocarboné saturé ou insaturé de 5 à 8 atomes de carbone, ou
R³, R⁴ et R⁵, ou R², R³ et R⁴ forment ensemble avec le noyau phényle auquel ils sont liés un noyau polycyclique à 3 cycles condensés, pour la synthèse de substrats d'enzyme.

13. Utilisation d'un dérivé d'aminocoumarine de formule : 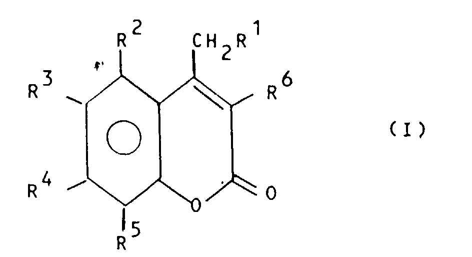 dans laquelle
1°) R¹ et R⁶ sont tels que définis dans la revendication 1,
2°) l'un des R², R³, R⁴ et R⁵ représente NHR⁹ ou NR⁷R⁹ avec R⁷ et R⁹ ayant les significations données dans la revendication 1, et
3°) les autres R², R³, R⁴ et R⁵ qui peuvent être identiques ou différents, représentent
- un atome d'hydrogène, ou
- un groupe alkyle ou alcoxy, non substitué ou substitué par au moins un atome d'halogène, ou
R² et R³, R³ et R⁴ ou R⁴ et R⁵ forment ensemble un cycle hydrocarboné saturé ou insaturé de 5 à 8 atomes de carbone, ou
R³, R⁴ et R⁵, ou R², R³ et R⁴ forment ensemble avec le noyau phényle auquel ils sont liés un noyau polycyclique à 3 cycles condensés, comme substrat d'enzyme.

## Patentansprüche

1. Aminocumarin-Derivat mit der Formel 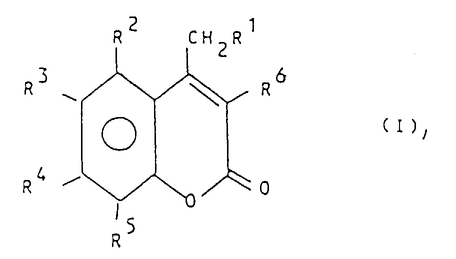 in welcher
R¹ einen Rest bedeutet, der aus geradkettigen oder verzweigten C₄- bis C₁₇-Alkylresten, C₃- bis C₁₀-Cycloalkylresten und C₆- bis C₁₄-Arylresten ausgewählt ist, wobei der Alkyl-, Cycloalkyl- oder Arylrest gegebenenfalls durch mindestens
- ein Halogenatom substituiert ist,
einer der Reste R², R³, R⁴ und R⁵
- NH₂,
- NHR⁷,
- NH-COOR⁷,
- N(R⁸)COOR⁷,
- NHR⁹ oder
- NR⁷R⁹
bedeutet, wobei R⁷ und R⁸ gegebenenfalls verschieden sind und einen C₁- bis C₁₇-Alkylrest oder einen C₆- bis C₁₄-Arylrest bedeuten oder zusammen einen gesättigten oder ungesättigten Kohlenwasserstoffcyclus bilden, der gegebenenfalls ein aus O, S und N ausgewähltes Heteroatom enthält, und R⁹ einen Acylrest bedeutet, der von einer aus Aminosäuren, Peptiden, Carbonsäuren und Fettsäuren ausgewählten Verbindung abgeleitet ist, wobei die Aminogruppe der Aminosäure oder des Peptids gegebenenfalls geschützt und gegebenenfalls versalzt ist,
die anderen, gegebenenfalls verschiedenen Reste R², R³, R⁴ und R⁵
- ein Wasserstoffatom oder
- einen Alkyl- oder Alkoxyrest bedeuten, der gegebenenfalls durch mindestens ein Halogenatom substituiert ist, oder R² und R³, R³ und R⁴ oder R⁴ und R⁵ zusammen einen gesättigten oder ungesättigten Kohlenwasserstoffcyclus mit 5 bis 8 Kohlenstoffatomen bilden oder R³, R⁴ und R⁵ oder R², R³ und R⁴ zusammen mit dem Phenylkern, an den sie gebunden sind, einen polycyclischen Kern mit 3 kondensierten Ringen bilden und
R⁶
- ein Wasserstoffatom oder
- einen aus C₁- bis C₁₇-Alkylresten, C₆- bis C₁₄-Arylresten und C₃- bis C₁₀-Cycloalkylresten ausgewählten Rest, wobei der Alkyl-, Aryl- oder Cycloalkylrest gegebenenfalls durch mindestens ein Halogenatom substituiert ist, und dessen Säureadditionssalze bedeutet.

2. Derivat nach Anspruch 1, **dadurch gekennzeichnet, daß** R¹ einen Alkylrest mit 4 bis 8 Kohlenstoffatomen bedeutet.

3. Derivat nach Anspruch 1, **dadurch gekennzeichnet, daß** R¹ den Butyl- oder Pentylrest bedeutet.

4. Derivat nach einem der Ansprüche 1 bis 3, **dadurch gekennzeichnet, daß** R⁴NH₂, NHR⁷, NHCOOR⁷, NHR⁹ oder NR⁷R⁹ bedeutet.

5. Derivat nach einem der Ansprüche 1 bis 3, **dadurch gekennzeichnet, daß** R⁴ NH₂, NHCOOR⁷ oder NHR⁷ bedeutet.

6. Derivat nach einem der Ansprüche 1 bis 3, **dadurch gekennzeichnet, daß** R⁴ NHCOOR⁷ bedeutet, wobei R⁷ für den Ethylrest steht.

7. Derivat nach einem der Ansprüche 1 bis 3, **dadurch gekennzeichnet, daß** R⁴ NHR⁹ oder NR⁷R⁹ bedeutet.

8. Derivat nach einem der Ansprüche 1 bis 3, **dadurch gekennzeichnet, daß** R⁴ NHR⁹ bedeutet, wobei R⁹ für den Acylrest steht, der von Alanin, das durch den Benzyloxycarbonyl-Rest geschützt oder mit Chlorwasserstoffsäure versalzt ist, abgeleitet ist.

9. Derivat nach einem der Ansprüche 1 bis 3, **dadurch gekennzeichnet, daß** R⁴ NHR⁹ bedeutet, wobei R⁹ für den Acylrest steht, der von Arginin oder von Arginin, das durch den tert.-Butyloxycarbonyl-Rest oder Benzyloxycarbonyl-Rest geschützt oder mit HCl bzw. CH₃COOH versalzt ist, abgeleitet ist.

10. Derivat nach einem der Ansprüche 1 bis 3, **dadurch gekennzeichnet, daß** R⁴ NHR⁹ bedeutet, wobei R⁹ für den Acylrest steht, der von dem Tripeptid Arg-Pro-Val abgeleitet ist, das durch den tert.-Butoxycarbonyl-Rest geschützt ist.

11. Derivat nach einem der Ansprüche 4 bis 10, **dadurch gekennzeichnet, daß** R², R³, R⁵ und R⁶ ein Wasserstoffatom bedeuten.

12. Verwendung eines Aminocumarin-Derivats mit der Formel 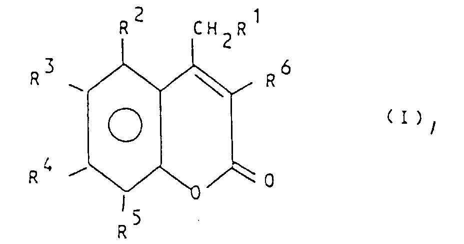 in welcher
R¹ und R⁶ wie im Anspruch 1 definiert sind und
einer der Reste R², R³, R⁴ und R⁵ -NH₂, -NHR⁷ oder
NHCOOR⁷ bedeutet, wobei R⁷ die im Anspruch 1 angegebene Bedeutung hat und die anderen Reste R², R³, R⁴ und R⁵, die gegebenenfalls verschieden sein können,
- ein Wasserstoffatom oder
- einen Alkyl- oder Alkoxyrest bedeuten, der gegebenenfalls durch mindestens einen aus den Halogenatomen ausgewählten Substituenten substituiert ist, oder R² und R³, R³ und R⁴ oder R⁴ und R⁵ zusammen einen gesättigten oder ungesättigten Kohlenwasserstoffcyclus mit 5 bis 8 Kohlenstoffatomen bilden oder R³, R⁴ und R⁵ oder R², R³ und R⁴ zusammen mit dem Phenylkern, an den sie gebunden sind, einen polycyclischen Kern mit 3 kondensierten Ringen bilden, für die Synthese von Enzymsubstraten.

13. Verwendung eines Aminocumarin-Derivats mit der Formel 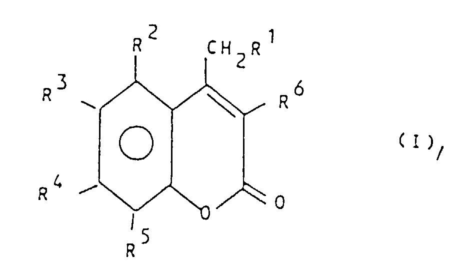 in welcher
R¹ und R⁶ wie im Anspruch 1 definiert sind,
einer der Reste R², R³, R⁴ und R⁵ NHR⁹ oder NR⁷R⁹ bedeutet, wobei R⁷ und
R⁹ die im Anspruch 1 angegebenen Bedeutungen haben, und
- die anderen Reste R², R³, R⁴ und R⁵, die gegebenenfalls verschieden sein können,
- ein Wasserstoffatom oder
- einen Alkyl- oder Alkoxyrest bedeuten, der gegebenenfalls durch mindestens ein Halogenatom substituiert ist, oder R² und R³, R³ und R⁴ oder R⁴ und R⁵ zusammen einen gesättigten oder ungesättigten Kohlenwasserstoffcyclus mit 5 bis 8 Kohlenstoffatomen bilden oder R³, R⁴ und R⁵ oder R², R³ und R⁴ zusammen mit dem Phenylkern, an den sie gebunden sind, einen polycyclischen Kern mit 3 kondensierten Ringen bilden, als Enzymsubstrat.

## Claims

1. Aminocoumarin derivative corresponding to the formula: 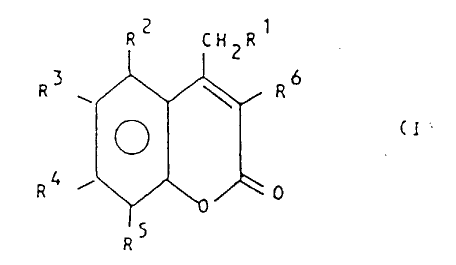 in which
1) R¹ represents a group chosen from linear or branched C₄ to C₁₇ alkyl groups, C₃ to C₁₀ cycloalkyl groups and C₆ to C₁₄ aryl groups, the alkyl, cycloalkyl or aryl group being unsubstituted or substituted by at least
- one halogen atom,
2) one of the R², R³, R⁴ and R⁵ groups represents
- NH₂
- NHR⁷
- NH-COOR⁷
- N(R⁸)COOR⁷
- NHR⁹, or
- NR⁷R⁹
with R⁷ and R⁸, which are identical or different, representing a C₁ to C₁₇ alkyl group or a C₆ to C₁₄ aryl group or with R⁷ and R⁸ together forming a saturated or unsaturated hydrocarbon ring, optionally containing a heteroatom chosen from 0, S and N, and R⁹ representing an acyl group derived from a compound chosen from amino acids, peptides, carboxylic acids and fatty acids, the amine group of the amino acid or of the peptide being protected or unprotected and salified or non-salified;
3) the other R², R³, R⁴ and R⁵ groups, which can be identical or different, represent
- a hydrogen atom, or
- an alkyl or alkoxy group which is unsubstituted or substituted by at least one halogen atom, or
R² and R³, R³ and R⁴ or R⁴ and R⁵ together form a saturated or unsaturated hydrocarbon ring containing 5 to 8 carbon atoms, or
R³, R⁴ and R⁵ or R², R³ and R⁴ form, together with the phenyl nucleus to which they are bonded, a polycyclic nucleus containing 3 condensed rings; and
4) R⁶ represents
- a hydrogen atom, or
- a group chosen from C₁ to C₁₇ alkyl, C₆ to C₁₄ aryl and C₃ to C₁₀ cycloalkyl groups, the alkyl, aryl or cycloalkyl group being unsubstituted or substituted by at least one halogen atom, and its addition salts with acids.

2. Derivative according to Claim 1, characterized in that R¹ represents an alkyl group containing 4 to 8 carbon atoms.

3. Derivative according to Claim 1, characterized in that R¹ represents the butyl or pentyl group.

4. Derivative according to any one of Claims 1 to 3, characterized in that R⁴ represents NH₂, NHR⁷, NHCOOR⁷, NHR⁹ or NR⁷R⁹.

5. Derivative according to any one of Claims 1 to 3, characterized in that R⁴ represents NH₂, NHCOOR⁷ or NHR⁷.

6. Derivative according to any one of Claims 1 to 3, characterized in that R⁴ represents NHCOOR⁷ with R⁷ representing the ethyl group.

7. Derivative according to any one of Claims 1 to 3, characterized in that R⁴ represents NHR⁹ or NR⁷R⁹.

8. Derivative according to any one of Claims 1 to 3, characterized in that R⁴ represents NHR⁹ with R⁹ representing the acyl group derived from alanine protected by the benzyloxycarbonyl group or salified by hydrochloric acid.

9. Derivative according to any one of Claims 1 to 3, characterized in that R⁴ represents NHR⁹ with R⁹ representing the acyl group derived from arginine or from arginine protected by the t-butyloxycarbonyl or benzyloxycarbonyl group or salified by HCl or CH₃COOH.

10. Derivative according to any one of Claims 1 to 3, characterized in that R⁴ represents NHR⁹ with R⁹ representing the acyl group derived from the tripeptide -Arg-Pro-Val protected by the t-butoxycarbonyl group.

11. Derivative according to any one of Claims 4 to 10, characterized in that R², R³, R⁵ and R⁶ represent a hydrogen atom.

12. Use of an aminocoumarin derivative of formula: 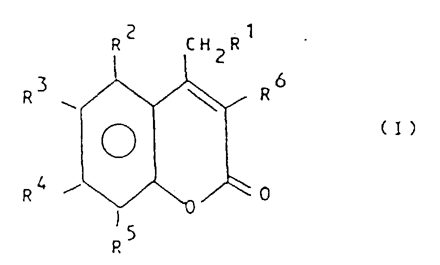 in which
1) R¹ and R⁶ are as defined in Claim 1,
2) one of the R², R³, R⁴ and R⁵ groups represents -NH₂, -NHR⁷ or -NHCOOR⁷ with R⁷ having the meaning given in Claim 1 and the other R², R³, R⁴ and R⁵ groups, which can be identical or different, represent
- a hydrogen atom, or
- an alkyl or alkoxy group which is unsubstituted or substituted by at least one substituent chosen from halogen atoms, or
R² and R³, R³ and R⁴ or R⁴ and R⁵ together form a saturated or unsaturated hydrocarbon ring containing 5 to 8 carbon atoms, or
R³, R⁴ and R⁵ or R², R³ and R⁴ form, together with the phenyl nucleus to which they are bonded, a polycyclic nucleus containing 3 condensed rings, for the synthesis of enzyme substrates.

13. Use of an aminocoumarin derivative of formula: 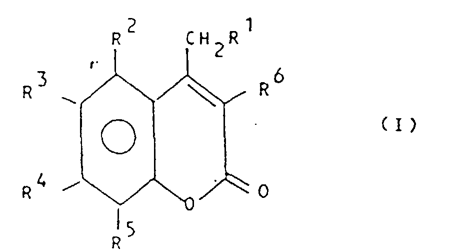 in which
1) R¹ and R⁶ are as defined in Claim 1,
2) one of the R², R³, R⁴ and R⁵ groups represents NHR⁹ or NR⁷R⁹ with R⁷ and R⁹ having the meanings given in Claim 1, and
3) the other R², R³, R⁴ and R⁵ groups, which can be identical or different, represent
- a hydrogen atom, or
- an alkyl or alkoxy group which is unsubstituted or substituted by at least one halogen atom, or
R² and R³, R³ and R⁴ or R⁴ and R⁵ together form a saturated or unsaturated hydrocarbon ring containing 5 to 8 carbon atoms, or
R³, R⁴ and R⁵ or R², R³ and R⁴ form, together with the phenyl nucleus to which they are bonded, a polycyclic nucleus containing 3 condensed rings, as enzyme substrate.
